# EUROPEAN PATENT APPLICATION

(11) **EP 3 884 946 A1**
(43) Date of publication of application: **29.09.2021**
(21) Application number: 20305327.7
(22) Date of filing: 25.03.2020
(51) Int. Cl.: A61K 31/47, A61K 31/7012, A61P 31/14

(54) **COMPOUNDS FOR TREATING OR PREVENTING A CORONAVIRIDAE INFECTION & METHODS AND USES FOR ASSESSING THE OCCURRENCE OF A CORONAVIRIDAE INFECTION**

(71) Applicant: ABIVAX, 75008 Paris (FR)
(72) Inventor: TAZI, Jamal, 34380 CLAPIERS (FR); EHRLICH, Hartmut, 75016 PARIS (FR); POULETTY, Philippe, 75005 PARIS (FR)
(74) Representative: Nony

(57) **Abstract**

COMPOUNDS FOR TREATING OR PREVENTING A CORONAVIRIDAE INFECTION & METHODS AND USES FOR ASSESSING THE OCCURENCE OF A CORONAVIRIDAE INFECTION

## Description

The present invention relates to the treatment or prevention of a *Coronaviridae* infection ; in particular a *Coronaviridae* infection in humans.

In particular, the invention relates to compounds, pharmaceutical compositions and medicaments for treating and/or preventing a *Coronaviridae* infection.

The invention further relates to uses and methods for assessing a *Coronaviridae* infection or the efficacy of a treatment of such infections.

### BACKGROUND

Viruses are one of the major causes of diseases around the world. Viruses are generally defined as small, non-living, infectious agents that replicate only within living cells, as they do not possess a completely autonomous replication mechanism. Although diverse in shape and size, they typically consist of a virus particle (known as a "virion"), made from a protein coat which comprises at least one nucleic acid molecule and optionally, depending on the type of virus, one or more proteins or nucleoproteins.

Even though their replication cycle varies greatly between species, it is generally recognized that the life cycle of viruses includes six basic steps: attachment, penetration, uncoating, replication, assembly and release.

Depending on the nature of the targeted virus, therapeutic molecules have been designed which may interfere with one or more of those mechanisms.

Among those, the replication step involves not only the multiplication of the viral genome, but also the synthesis of viral messenger RNA, of viral protein synthesis, and the modulation or use of the transcription or translation machinery of the host. However, it is also clear that the type of genome (single-stranded, double-stranded, RNA, DNA...) characterizes dramatically this replication step. For instance, most DNA viruses assemble in the nucleus while most RNA viruses develop solely in the cytoplasm. Also, there is increasing evidence that single-stranded RNA viruses use the host RNA splicing and maturation machinery.

Accordingly, and considering the implications of a given type of genome in the replication step, the Baltimore classification of viruses was developed. This classification clusters viruses into families (or *"groups"*) depending on their type of genome. The present virus classification, as in 2018, comprises seven different groups:
- Group I: double-stranded DNA viruses (dsDNA);
- Group II: single-stranded DNA viruses (ssDNA);
- Group III: double-stranded RNA viruses (dsRNA);
- Group IV: (+)strand or sense RNA viruses ((+)ssRNA);
- Group V: (-)strand or antisense RNA viruses ((-)ssRNA);
- Group VI: single-stranded RNA viruses having DNA intermediates (ssRNA-RT);
- Group VII: double-stranded DNA viruses having RNA intermediates (dsDNA-RT).

There are few cures for diseases caused by RNA virus infections, in particular single-stranded RNA viruses, and more specifically RNA virus infections from viruses belonging to group IV of the Baltimore classification.

Strikingly, an acute respiratory disease was recently found to be caused by a novel coronavirus (SARS-CoV-2, previously known as 2019-nCoV), also referred herein as the coronavirus disease 2019 (COVID-19), which belongs to the *Coronaviridae* family and which part of the group IV of the Baltimore classification.

This coronavirus shows sustained human-to-human transmission, along with many exported cases across the globe. World Health Organization (WHO) has officially declared the COVID-19 pandemic as a public health emergency of international concern. The novel coronavirus uses the same receptor, angiotensin-converting enzyme 2 (ACE2) as that for Severe Acute Respiratory Syndrome (SARS)-CoV, and mainly spreads through the respiratory tract. The elderly and people with underlying diseases are susceptible to infection and prone to serious outcomes, which may be associated with acute respiratory distress syndrome (ARDS) and cytokine storm.

Three strategies for therapy are currently explored :
(i) to limit the spreading of the COVID-19 infection by blocking the replication of the virus. This can be achieved through inhibition of RNA dependent polymerase (RdRp) of the virus or by preventing the entry of the virus in pulmonary target cells ;
(ii) to dampen the inflammation of the pulmonary tracts ;
(iii) to promote a vaccination strategy.

Antiviral drugs for managing infections with human coronaviruses are not yet approved, posing a serious challenge to current global efforts aimed at containing the outbreak of COVID-19. Some companies are testing several combinations in clinical trials to find antiviral compounds, mainly nucleoside analogues, in order to block the RNA-dependent RNA polymerase (RdRp) of the virus, and anti-proteases in order to block the entry of the virus. Other strategies involve biologics and the combination of drugs, in order to block the inflammation.

The response to each of these therapies is highly uncertain at the moment and requires other options to be explored.

WO2014111892 teaches the use of miR-124 as a biomarker of an HIV infection.

WO2016135052 and WO2016135055 teach the use of quinoline derivatives and metabolites thereof for treating or preventing viral infections, including HIV infections.

Indeed, originally developed as an inhibitor of HIV replication and HIV reservoir reduction, a set of quinoline derivatives including the ABX464 compound has now been found to bind to the Cap Binding Complex (CBC) at the interface between the two "20" and "80" subunits of a large complex that regulates splicing and export of mRNA from the nucleus. The active metabolite of ABX464, a N-glucuronidated form referred herein as ABX464-N-Glu, also binds to the CBC complex. The ABX464-CBC interaction has been shown to strengthen the RNA quality control of HIV-RNA biogenesis, thus preventing the production of unspliced HIV RNA and to reduce reservoirs of HIV infected patients.

While capable of altering directly the splicing of a small number of genes in cells, the examination of the effects of ABX464 on the microRNAs profile has shown that the expression of a single miRNA was significantly increased by ABX464: miR-124.

WO2020011810 now further teaches the use of quinoline derivatives for treating or preventing a RNA virus infection, and more particularly RNA viruses belonging to the group IV of the Baltimore classification, to which the *Coronaviridae* family belongs.

However there remains a need for novel compounds for treating or preventing a RNA virus infection, and especially a *Coronaviridae* infection.

The invention has for purpose to meet the above-mentioned needs.

### DETAILED DESCRIPTION OF THE INVENTION

The inventors have surprisingly found that dynamin 2 (DNM2), which is a GTPase responsible for vesicle scission, is a target of miR-124, especially in the context of a *Coronaviridae* infection.

Dynamin-2 is a well-known pleiotropic GTPase which is involved in many membrane-remodeling events, including membrane scission during vesicle budding from the plasma or Golgi membranes, ynaptic vesicle recycling, post-synaptic receptor internalization, neurosecretion, and neuronal process extension.

Dynamin-2 on its own is now further shown to be a therapeutic target for treating or preventing a *Coronaviridae* infection, through the development of so-called dynamin inhibitors (i.e. dynamin-2 inhibitors), such as phenothiazine and phenothiazine-derived drugs.

Indeed, although compounds belonging to the phenothiazine class, such as chlorpromazine, are known to exert anti-prion effects, applications of this particular class of active agents have not yet been reported for the treatment or prevention of a *Coronaviridae* infection.

While, on one side, ABX464 is known to decrease inflammation, it is now further demonstrated that uncontrolled pulmonary inflammation can be critical for the prognosis and death of COVID-19 infections. Indeed, many COVID-19 patients develop acute respiratory distress syndrome (ARDS), which leads to pulmonary edema and lung failure. Without wishing to be bound by the theory, the inventors thus propose that elevated pro inflammatory cytokines involved in Th17 responses in COVID-19 infected patients may be the cause of vascular permeability and leakage.

Hence, the inventors propose that attenuation of Th17 proliferation by ABX464, or its N-glucuronide metabolite (ABX646-N-Glu), may treat or prevent a *Coronaviridae* infection, and especially, the severe acute respiratory syndrome caused by COVID-19 infection.

The inventors also propose that the dual ability of ABX464, or its N-glucuronide metabolite, to dampen inflammation and reducing viral load by controlling viral RNA biogenesis or viral particle endocytosis have applicability for the treatment or prevention of *Coronaviridae,* including the COVID-19.

Indeed, COVID19 contain a non-segmented, positive-sense RNA genome of ∼30 kb. The genome contains a 5' cap structure along with a 3' poly (A) tail, allowing it to act as a mRNA for translation of the replicase polyproteins. The 5' cap can be recognized by eIF4E and/or CBC complex to initiate translation and/or RNA quality control, respectively. Like in HIV, CBC-ABX464 may favour the RNA quality control of COVID-19 RNA genome and block the production of RdRp polymerase thereby interfering with viral replication.

In addition, it is proposed herein that miR-124 up-regulation may directly interfere with the entry of the COVID-19 to tracheobronchial tissue. As virions, after binding to ACE2 receptor and the action of serine protease TMPRSS2 for S protein priming, indeed require clathrin-mediated endocytosis for successful entry, and subsequent vesicle scission by dynamin 2, which is a direct target of miR-124.

Thus, ABX464 and its N-glucuronide metabolite may tackle both the *Coronaviridae* infection and the induced inflammation. Advantageously, the safety profile of ABX-464 is also very favourable with no drug related serious adverse reactions.

Advantageously, ABX464 and its N-glucuronide metabolite may be considered either alone or in combination with any other active agent, in particular any other dynamin inhibitor, especially any dynamin-2 inhibitor, which is reported herein, for use in the treatment of prevention of a *Coronaviridae* infection.

According to a first main embodiment, the invention relates to a compound of formula (I) 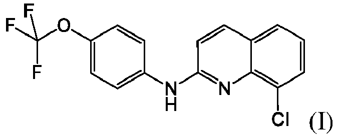 or any one of its or any one of its prodrugs or any one of its pharmaceutically acceptable salts, for use in a method for treating or preventing a *Coronaviridae* infection. As used herein, the term *"**ABX464**"* refers to such compound of formula (I) or any one of its or any one of its prodrugs or any one of its pharmaceutically acceptable salts.

In particular, the invention relates to a compound of formula (I) 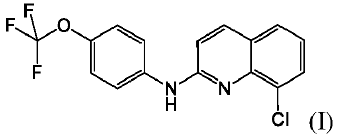 or any one of its pharmaceutically acceptable salts, for use in a method for treating or preventing a *Coronaviridae* infection.

According to a second main embodiment, the invention relates to a compound of formula (II) 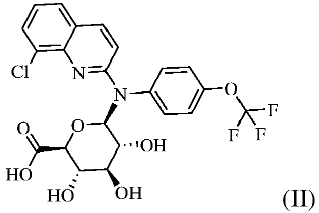 or any one of its or any one of its prodrugs or any one of its pharmaceutically acceptable salts for use in a method for treating or preventing a *Coronaviridae* infection. As used herein, the term *"**ABX464-N-Glu**"* refers to such compound of formula (II) or any one of its or any one of its prodrugs or any one of its pharmaceutically acceptable salts.

In particular, the invention relates to a compound of formula (II) 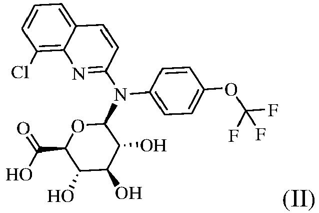 or any one of its pharmaceutically acceptable salts for use in a method for treating or preventing a *Coronaviridae* infection.

According to a third main embodiment, the invention relates to a pharmaceutical composition comprising a compound of formula (I) or (II) as defined above or any one of its prodrugs or any one of its pharmaceutically acceptable salts, and at least one pharmaceutically acceptable excipient, for use in a method for treating or preventing a *Coronaviridae* infection.

According to a fourth main embodiment, the invention relates to a medicament comprising a compound a compound of formula (I) or (II) as defined above, for use in a method for treating or preventing a *Coronaviridae* infection.

According to a fifth main embodiment, the invention relates to an *in vitro* or *ex vivo* use of at least one miRNA, said at least one miRNA being miR-124, as a biomarker of a *Coronaviridae* infection, or of an efficacy of a therapeutic treatment of said *Coronaviridae* infection.

According to a sixth main embodiment, the invention relates to an *in vitro* or *ex vivo* method for assessing a *Coronaviridae* infection in a patient presumed to be infected with a virus, comprising at least the steps of:
a- measuring a presence or an expression level of at least one miRNA, said at least one miRNA being miR-124, in a biological sample previously obtained from said patient; and
b- comparing said presence or expression level to a control reference value, wherein a modulated presence or level of expression of said miRNA relative to said control reference value is indicative of a *Coronaviridae* infection.

According to a seventh main embodiment, the invention relates to a dynamin inhibitor for use in a method for treating or preventing a *Coronaviridae* infection; in particular for reducing a *Coronaviridae* viral load. In particular, the *Coronaviridae* may be COVID-19 or any one of its mutants.

### Definitions

As used herein, the term "patient" refers to either an animal, such as a valuable animal for breeding, company or preservation purposes, or preferably a human or a human child, which is afflicted with, or has the potential to be afflicted with one or more diseases and conditions described herein.

In particular, as used in the present application, the term "patient" refers to a mammal such as a rodent, cat, dog, primate or human, preferably said subject is a human and also extends to birds.

The identification of those patients who are in need of treatment of herein-described diseases and conditions is well within the ability and knowledge of one skilled in the art. A veterinarian or a physician skilled in the art can readily identify, by the use of clinical tests, physical examination, medical/family history or biological and diagnostic tests, those patients who are in need of such treatment.

In the context of the invention, the term "treating" or "treatment", as used herein, means reversing, alleviating, inhibiting the progress of, or preventing the disease resulting from RNA virus infection, and more particularly RNA virus infection from group IV or V, or one or more symptoms of such disease.

As used herein, an "effective amount" refers to an amount of a compound of the present invention which is effective in preventing, reducing, eliminating, treating or controlling the symptoms of the herein-described diseases and conditions, *i.e.* RNA virus infection, and more particularly RNA virus infection from group IV and V. The term "controlling" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment.

The term *"effective amount"* includes *"prophylaxis-effective amount"* as well as *"treatment-effective amount".*

The term "preventing", as used herein, means reducing the risk of onset or slowing the occurrence of a given phenomenon, namely in the present invention, a disease resulting from a RNA virus infection, and more particularly a RNA virus infection from group IV or V.

As used herein, « *preventing* » also encompasses « *reducing the likelihood of occurrence* » or « *reducing the likelihood of reoccurrence ».*

The term *"prophylaxis-effective amount"* refers to a concentration of compound of this invention that is effective in inhibiting, preventing, decreasing the likelihood of the disease by RNA viruses, and more particularly by a RNA virus from group IV or V of the Baltimore classification, or preventing the RNA virus infection and in particular a RNA virus infection from group IV or preventing the delayed onset of the disease by the RNA virus, and more particularly by a RNA virus from group IV, when administered before infection, *i.e.* before, during and/or slightly after the exposure period to the RNA virus, and in particular to the RNA virus from group IV.

Likewise, the term *"treatment-effective amount"* refers to a concentration of compound that is effective in treating the RNA virus infection, *e.g.* leads to a reduction in RNA viral infection, following examination when administered after infection has occurred.

As used herein, the term *"pharmaceutically acceptable"* refers to those compounds, materials, excipients, compositions or dosage forms which are, within the scope of sound medical judgment, suitable for contact with the tissues of human beings and animals without excessive toxicity, irritation, allergic response or other problem complications commensurate with a reasonable benefit/risk ratio.

As used herein, the term *"pharmaceutically acceptable salt"* refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge et al., describe pharmaceutically acceptable salts in detail in J. Pharmaceutical Sciences, 1977, 66, 1-19, incorporated herein by reference. Pharmaceutically acceptable salts of the compounds of this invention include those derived from suitable inorganic and organic acids and bases.

The term *"pharmaceutically acceptable carrier, adjuvant, or vehicle"* may refer to any pharmacutically acceptable excipient, such as a non- toxic carrier, adjuvant, or vehicle that does not destroy the pharmacological activity of the compound with which it is formulated. Pharmaceutically acceptable carriers, adjuvants or vehicles that may be used in the compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

A *"biological sample"* suitable for the present invention can be a biological fluid, such as a blood, a plasma, or a serum, a saliva, an interstitial, fluid, or a urine sample; a cell sample, such as a cell culture, a cell line, or a PBMC sample, a tissue biopsy, such as an oral tissue, a gastrointestinal tissue, a skin, an oral mucosa sample, a pharyngeal, tracheal, bronchoalveolar sample, or a plurality of samples from a clinical trial.

A biological sample can be a crude sample, or can be purified to various degrees prior to storage, processing, or measurement. In some embodiments, a biological sample is selected from the group consisting of a biological tissue sample, a whole blood sample, a swab sample, a plasma sample, a serum sample, a saliva sample, a vaginal fluid sample, a sperm sample, a pharyngeal fluid sample, a synovial sample, a bronchial or pleural fluid sample, a fecal fluid sample, a cerebrospinal fluid sample, a lacrymal fluid sample and a tissue culture supernatant sample.

As used herein, the term *"miR-124"* refers to either one of the 9 haplotypes of miR-124 precursors have been identified so far (Guo et al., PLoS ONE, 2009, 4(11):e7944), from which 3 are present in the Human, hsa-miR-124-1, hsa-miR-124-2 and hsa-miR-124-3. The miR-124 microRNA precursor is a small non-coding RNA molecule. The mature ∼21 nucleotide microRNAs are processed from hairpin precursor sequences by the Dicer enzyme. The mature sequences are reported in WO2014111892**.**

As used herein, an *"viral infection or related condition"* refers to an infection of condition related to a virus, more particularly said virus having a RNA genome, and especially a RNA virus belonging to group IV according to the Baltimore classification. Viruses may be further classified in distinct families, orders and genus.

For reference, the content of the *"Baltimore classification"* which is reported herein further references to the virus taxonomy as set forth in the database of the International Committee of Taxonomy of Viruses (ICTV) as available online on March 20, 2020 (Email ratification February 2019 & MSL #34) at https://talk.ictvonline.org/taxonomy/. This taxonomy is incorporated herein in its entirety.

Accordingly, this classification clusters viruses into families (or *"groups"*) depending on their type of genome. The present virus classification, as in 2018, comprises seven different groups:
- Group I: double-stranded DNA viruses (dsDNA);
- Group II: single-stranded DNA viruses (ssDNA);
- Group III: double-stranded RNA viruses (dsRNA);
- Group IV: (+)strand or sense RNA viruses ((+)ssRNA);
- Group V: (-)strand or antisense RNA viruses ((-)ssRNA);
- Group VI: single-stranded RNA viruses having DNA intermediates (ssRNA-RT);
- Group VII: double-stranded DNA viruses having RNA intermediates (dsDNA-RT).

The compounds of formula (I) and (II), and their corresponding prodrugs, or anyone of their pharmaceutical salts are reported herein for the treatment or prevention of infections against the following viruses: HSV, CMV, EBV, Adenoviruses, Pox viruses, HPV (human papilloma virus), Parvovirus, Reoviruses, Hepatitis A virus, Rubella virus, Hepatitis C virus (HCV), Hepatitis E virus, Dengue virus, Chikungunya virus, Zika virus, Enteroviruses, Rhinoviruses, poliovirus, foot-and-mouth virus, yellow fever virus, Paramyxoviruses, Influenza viruses Retroviruses including HTLV-1, HTLV-2, HIV and Hepatitis B (HBV), due to their reliance on Dynamin 2-mediated endocytosis.

Unless instructed otherwise, all the disclosed compounds are specifically considered herein for the treatment or prevention of *Coronaviridae,* which may thus refer indifferently to any member of the said *Coronaviridae* family in the sense of the Baltimore convention, although particular selections of viruses will be considered hereafter as preferred embodiments.

The same applies to the uses & methods which are considered as part of the present invention, including iomarkers uses and methods for assessing a *Coronaviridae infection* or the efficacy of a particular therapy directed against said *Coronaviridae* infection.

As used herein, the term *"Coronaviridae*" refers to the corresponding family of RNA viruses belonging to the group IV of the Baltimore classification, which is it iself par of the *Cornidovirineae* suborder and of the *Nidovirales* Order. The *Coronaviridae* family includes both the *Letovirinae* and *Orthocoronavirinae* subfamilies.

As used herein, the term *"Letovirinae* " refers to the corresponding family of the Baltimore classification, which includes the *Alphaletovirus* genus, the *Milecovirus* subgenus, which includes (in a non-exhaustive manner) the *Microhyla letovirus 1* species.

As used herein, the term *"Orthocoronavirinae"* refers to the corresponding family of the Baltimore classification, which includes the *Alphacoronavirus, Betacoronavirus, Deltacoronavirus,* and *Gammacoronavirus* genus.

As used herein, the term *"Alphacoronavirus*" refers to the corresponding family of the Baltimore classification, which includes the *Colacovirus, Decacovirus, Duvinacovirus, Luchacovirus, Minacovirus, Minunacovirus, Myotacovirus, Myctacovirus, Pedacovirus, Rhinacovirus, Setracovirus,* and *Tegacovirus* subgenus. In a non-exhaustive manner, this includes the following species: *Bat coronavirus CDPHE15, Bat coronavirus HKU10, Rhinolophus ferrumequinum alphacoronavirus HuB-2013, Human coronavirus 229E, Lucheng Rn rat coronavirus, Ferret coronavirus, Mink coronavirus 1, Miniopterus bat coronavirus 1, Miniopterus bat coronavirus HKU8, Myotis ricketti alphacoronavirus Sax-2011, Nyctalus velutinus alphacoronavirus SC-2013, Porcine epidemic diarrhea virus, Scotophilus bat coronavirus 512, Rhinolophus bat coronavirus HKU2, Human coronavirus NL63, NL63-related bat coronavirus strain BtKYNL63-9b, Alphacoronavirus 1.*

As used herein, the term *"Betacoronavirus"* refers to the corresponding family of the Baltimore classification, which includes the *Embecovirus, Hibecovirus, Merbecovirus, Nobecovirus,* and *Sarbecovirus* subgenus. In a non-exhaustive manner, this includes the following species: *Betacoronavirus 1, China Rattus coronavirus HKU24, Human coronavirus HKU1, Murine coronavirus, Bat Hp-betacoronavirus Zhejiang2013, Hedgehog coronavirus 1, Middle East respiratory syndrome-related coronavirus, Pipistrellus bat coronavirus HKU5, Tylonycteris bat coronavirus HKU4, Hedgehog coronavirus 1, Middle East respiratory syndrome-related coronavirus, Pipistrellus bat coronavirus HKU5, Tylonycteris bat coronavirus HKU4, Rousettus bat coronavirus GCCDC1, Rousettus bat coronavirus HKU9, Severe acute respiratory syndrome-related coronavirus.*

As used herein, the term *"Severe acute respiratory syndrome-related coronavirus",* or SARS virus, includes, in a non-exhaustive manner, the *SARS-CoV, SARSr-CoV WIV1, SARSr-CoV HKU3, SARSr-CoV RP3,* and *SARS-CoV-2; including COVID-19 and their mutants.*

As used herein, the term *"Deltacoronavirus"* refers to the corresponding family of the Baltimore classification, which includes the *Andecovirus, Buldecovirus, Herdecovirus,* and *Moordecovirus* subgenus. In a non-exhaustive manner, this includes the following species: *Wigeon coronavirus HKU20, Bulbul coronavirus HKU11, Coronavirus HKU15, Munia coronavirus HKU13, White-eye coronavirus HKU16, Night heron coronavirus HKU19, Common moorhen coronavirus HKU21.*

As used herein, the term *"Gammacoronavirus"* refers to the corresponding family of the Baltimore classification, which includes the *Cegacovirus* and *Igacovirus* subgenus. In a non-exhaustive manner, this includes the following species: *Beluga whale coronavirus SW1* and *Avian coronavirus.*

As used herein, the term *"a phenothiazine"* refers to an heterocyclic compound of the thiazine class, including phenothiazine and phenothiazine-derivatives, in particular those which are characterized by the following formula 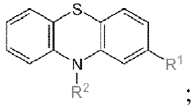 wherein
R¹ can be any chemical substituent, in particular any chemical substituent selected from: a halogen, an alkyl group, a substituted alkyl group, an alkoxy group, a substituted alkoxy group, a thioether or an acetyl group
R² can be selected from acyclic groups, piperidine-derived groups and piperazine-derived groups.

### Compounds for use

According to a first main embodiment, the invention relates to a compound of formula (I) 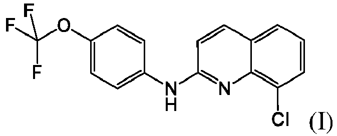 or any one of its or any one of its prodrugs or any one of its pharmaceutically acceptable salts, for use in a method for treating or preventing a *Coronaviridae* infection.

Said compound is 8-Chloro-N-(4-(trifluoromethoxy)phenyl)quinolin-2-amine.

According to a second main embodiment, the invention relates to a compound of formula (II) 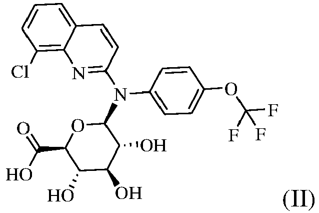 or any one of its or any one of its prodrugs or any one of its pharmaceutically acceptable salts for use in a method for treating or preventing a *Coronaviridae* infection.

The compounds of the present invention (of formula (I) or (II) can be prepared by conventional methods of organic synthesis practiced by those skilled in the art. The general reaction sequences outlined below represent a general method useful for preparing the compounds of the present invention and are not meant to be limiting in scope or utility.

The man skilled in the Art may, for instance, refer to the content of WO2016135052 and WO2016135055 for that matter.

The compounds of the invention may exist in the form of free bases or of addition salts with pharmaceutically acceptable acids.

In particular, « *Pharmaceutically acceptable salt thereof* » refers to salts which are formed from acid addition salts formed with inorganic acids (e.g. hydrochloric acid, hydrobromic acid, sulfuric acid, phosphoric acid, nitric acid, and the like), as well as salts formed with organic acids such as acetic acid, oxalic acid, tartaric acid, succinic acid, malic acid, fumaric acid, maleic acid, ascorbic acid, benzoic acid, tannic acid, palmoic acid, alginic acid, polyglutamic acid, naphthalene sulfonic acid, naphthalene disulfonic acid, and poly-galacturonic acid.

Suitable physiologically acceptable acid addition salts of compounds of formula (I) or (II), or prodrugs thereof, may include hydrobromide, tartrate, citrate, trifluoroacetate, ascorbate, hydrochloride, tosylate, triflate, maleate, mesylate, formate, acetate and fumarate.

The compounds of formula (I) or (II), and their prodrugs, or any of their pharmaceutically acceptable salts may form solvates or hydrates and the invention includes all such solvates and hydrates.

The terms *"hydrates"* and *"solvates"* simply mean that the compounds according to the invention can be in the form of a hydrate or solvate, *i.e.* combined or associated with one or more water or solvent molecules. This is only a chemical characteristic of such compounds, which can be applied for all organic compounds of this type.

Pharmaceutically acceptable salts of the compounds of this invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid, persulfuric acid, boric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzoate, edetate, gluceptate, bisulfate, borate, butyrate, camphorate, cyclopentaneproprionate, citrate, glycerophosphoric acid, nitric acid, cyclopentanepropionate, digluconate, dodecylsulfate, formate, acetate, fumarate, glucoheptonate, glycerophosphate, gluconate, hemisulfate, glucoheptonate, heptanoate, hexanoate, hydroiodide, lactobionate, lactate, laurate, lauryl sulfate, malate, maleate, malonate, mandelate, salicylate, disalicylate, picrate, mucate, , nicotinate, nitrate, oleate, oxalate, palmitate, pamoate, pectinate, persulfate, dodecylsulfate, 3-phenylpropionate, phosphate, pivalate, propionate, undecanoate stearate, succinate, bitartrate, sulfate, tartrate, trifluoroacetate, triflate, thiocyanate, undecanoate, valerate salts, pantothenate, dodecylsulfatesulfonate, in particular alkylsufonate such as methanesulfonate (or mesylate), esylate, edisylate, estolate, ethanesulfonate, 2- hydroxy-ethanesulfonate or arylsulfonate, such as 2-naphthalenesulfonate, napadisylate, napsylate, camphorsulfonate, benzenesulfonate (or besylate), p-toluenesulfonate (or tosylate), and the like.

In particular, the pharmaceutically acceptable salts are selected from the group consisting of:
- salts formed with inorganic acids such as hydrochloric acid, hydrobromic acid, phosphoric acid, sulfuric acid, persulfuric acid, boric acid and perchloric acid,
- salts formed with organic acids such as acetic acid, oxalic acid, maleic acid, tartaric acid, citric acid, succinic acid or malonic acid, and
- one salt selected from adipate, alginate, ascorbate, aspartate, benzoate, edetate, gluceptate, bisulfate, borate, butyrate, camphorate, cyclopentaneproprionate, citrate, glycerophosphoric acid, nitric acid, cyclopentanepropionate, digluconate, dodecylsulfate, formate, acetate, fumarate, glucoheptonate, glycerophosphate, gluconate, hemisulfate, glucoheptonate, heptanoate, hexanoate, hydroiodide, lactobionate, lactate, laurate, lauryl sulfate, malate, maleate, malonate, mandelate, salicylate, disalicylate, picrate, mucate, , nicotinate, nitrate, oleate, oxalate, palmitate, pamoate, pectinate, persulfate, dodecylsulfate, 3-phenylpropionate, phosphate, pivalate, propionate, undecanoate stearate, succinate, bitartrate, sulfate, tartrate, trifluoroacetate, triflate, thiocyanate, undecanoate, valerate salts, pantothenate, dodecylsulfate, sulfonate, in particular alkylsufonate such as methanesulfonate (or mesylate), esylate, edisylate, estolate, ethanesulfonate, 2- hydroxy-ethanesulfonate or arylsulfonate, such as 2-naphthalenesulfonate, napadisylate, napsylate, camphorsulfonate, benzenesulfonate and p-toluenesulfonate.

More particularly, the pharmaceutically acceptable salts are selected from sulfate, hydrobromide, citrate, trifluoroacetate, ascorbate, hydrochloride, tartrate, triflate, maleate, mesylate, formate, acetate, fumarate and sulfonate, in particular alkylsufonate or arylsulfonate, and more particularly mesylate, triflate, edisylate, besylate and tosylate.

According to one embodiment, ABX464 and its metabolites, and more particularly N-glucuronide metabolites of ABX464, including compound of formula (1) as defined above is in a salt form selected from lactate, oleate, oxalate, palmitate, stearate, valerate, butyrate, malonate, succinate, malate, benzoate, gluconate, lactobionate, pamoate, adipate, alginate, aspartate, camphorate, digluconate, heptanoate, hexanoate, laurate, nicotinate, pivalate, propionate, and the like, phosphate and the like, camphorsulfonate, 2-hydroxy-ethanesulfonate, esylate, napadisylate, and the like, perchloric acid, and the like, and is particularly selected from esylate and napadisylate, even more particularly is selected from anhydrous crystalline ABX464 hemi-napadisylate salt, anhydrous crystalline ABX464 esylate salt, and crystalline hemi-THF solvate of ABX464 hemi-napadisylate.

In some embodiments, the compound ABX464, or a pharmaceutically acceptable salt thereof, the compound ABX464, or a pharmaceutically acceptable salt thereof, is under a crystallized form. In some embodiments, a crystallized form of the compound ABX464, or a pharmaceutically acceptable salt thereof, has a melting point at 120.5°C (± 2°C).

In some embodiments, a crystallized form of the compound ABX464, or a pharmaceutically acceptable salt thereof, shows peaks in an x-ray powder diffractogram (XRPD) at angles 7.3, 14.6, 18.4, and 24.9. In some embodiments, a crystallized form of the compound ABX464, or a pharmaceutically acceptable salt thereof, shows one or more XRPD peaks at angles selected from 18.0, 24.2, 28.3, and 29.5. In some embodiments, a crystallized form of the compound ABX464, or a pharmaceutically acceptable salt thereof, shows one or more XRPD peaks at angles selected from 18.6, 22.3, 23.0, and 23.5.

According to a particular embodiment, the crystalline polymorphic form of 8-Chloro-N-(4-(trifluoromethoxy)phenyl)quinolin-2-amine is characterized by the following main peaks expressed as degree 2-Theta angles by a XRPD analysis: 7.3, 14.6, 23.5, and 28.4 (each time ±0.2) and may further show the following additional peaks expressed as degree 2-Theta angles: 12.1, 17.3, 18.4, 23.0; 24.2, 24.9, 27.4 and 29.1 (each time ±0.2) and even optionally further the following additional peaks expressed as degree 2-Theta angles: 13.7, 16.3, 16.9, 18.1, 22.4, and 29.6 (each time ±0.2).

According to one more particular embodiment, ABX464 is in a crystalline salt form selected from:
- anhydrous crystalline ABX464 hemi-napadisylate salt having a powder X-ray diffractogram displaying peaks expressed as degree 2-Theta angle at 9.8; 16.4; 18.2; 20.1; 21.2; 21.6; 23.5 and 26.3 (each time ±0.2), and optionally further shows the following additional peaks expressed as degree 2-Theta angle: 12.4; 13.1; 17.8; 20.9; 22.6; 24.5; 24.7; 25.2; and 25.9 (each time ±0.2); and even optionally further the following additional peaks expressed as degree 2-Theta angle: 8.8; 13.3; 15.1; 17.2; 17.5; 19.4; 19.5; and 19.8 (each time ±0.2) and/or having a single endotherm with an onset temperature of 269.0°C (±2°C);
- anhydrous crystalline ABX464 esylate salt having a powder X-ray diffractogram displaying peaks expressed as degree 2-Theta angle at 12.2; and 22.2 (each time ±0.2), and optionally further shows the following additional peaks expressed as degree 2-Theta angle: 6.2; 12.9; 13.1; 15.3; 16.3; 18.2; 18.6; 19.5; 20.0; and 20.7 (each time ±0.2); and even optionally further the following additional peaks expressed as degree 2-Theta angle: 10.1; 15.8; 17.7; 17.9; 20.3; and 21.4 (each time ±0.2), and/or having a single endotherm with an onset temperature of 108.0°C (±2°C); and
- crystalline hemi-THF solvate of ABX464 hemi-napadisylate salt having a powder X-ray diffractogram displaying peaks expressed as degree 2-Theta angle at 8.4; 12.3; 14.0; 19.2; 21.3; 22.6 and 24.6 (each time ±0.2), and optionally further shows the following additional peaks expressed as degree 2-Theta angle: 9.6; 13.0; 13.5; 14.8; 17.2; 17.8; 23.4; 24.1; 24.9 and 25.2 (each time ±0.2); and even optionally further the following additional peaks expressed as degree 2-Theta angle: 16.7; 18.1; 18.8; 19.5; 20.9 and 22.3 (each time ±0.2), and/or having a single endotherm with an onset temperature of 172.0°C (±2°C).

According to one embodiment, ABX464 and its metabolites, and more particularly N-glucuronide metabolites of ABX464, including compound of formula (1) as defined above is in a co-crystal form with a co-crystal selected from: L-Proline, Gentisic acid, Malonic acid and 4, 4'-Bipyridine.

According to one more particular embodiment, ABX464 is in a co-crystal form selected from:
- 8-Chloro-N-(4-(trifluoromethoxy)phenyl)quinolin-2-amine : L-Proline having a powder X-ray diffractogram displaying peaks expressed as degree 2-Theta angle at 16.5; 20.6; 21.4; and 22.1 (each time ±0.2), and which may optionally further show the following additional peaks expressed as degree 2-Theta angle: 11.0; 15.9; 18.3; and 19.4 (each time ±0.2); and even optionally further the following additional peaks expressed as degree 2-Theta angle 6.1; 12.2; 12.6; 13.3; 13.7; 15.4; 17.3 and 22.4 (each time ±0.2), optionally further characterized by a powder X-ray diffractogram and/or having a single endotherm with an onset temperature of 172.0°C (±2°C);
- 8-Chloro-N-(4-(trifluoromethoxy)phenyl)quinolin-2-amine : Gentisic acid having a powder X-ray diffractogram displaying peaks expressed as degree 2-Theta angle at 7.9; 14.0; 15.2; and 25.2 (each time ±0.2), and which may optionally further show the following additional peaks expressed as degree 2-Theta angle: 15.8; 16.9; 18.5; 19.9; 20.3; 23.0 and 24.7 (each time ±0.2); and even optionally further the following additional peaks expressed as degree 2-Theta angle: 7.6; 14.7; 16.1; 19.7; 21.6; 22.0; 22.3; 23.7; and 24.0 (each time ±0.2), optionally further characterized by a powder X-ray diffractogram and/or having a single endotherm with an onset temperature of 133.0°C (±2°C);
- 8-Chloro-N-(4-(trifluoromethoxy)phenyl)quinolin-2-amine : Malonic acid having a powder X-ray diffractogram displaying peaks expressed as degree 2-Theta angle at 9.5; 12.2; 15.8; 17.3; 19.7; 22.8; 24.8; and 25.6 (each time ±0.2), and which may optionally further show the following additional peaks expressed as degree 2-Theta angle: 19.0; 21.4; 24.6; 26.8; 27.6; and 29.9 (each time ±0.2); and even optionally further the following additional peaks expressed as degree 2-Theta angle 16.8; 17.8; 20.9; 23.8; 28.0; and 29.6 (each time ±0.2), optionally further characterized by a powder X-ray diffractogram and/or having a single endotherm with an onset temperature of 109.0°C (±2°C); and
- 8-Chloro-N-(4-(trifluoromethoxy)phenyl)quinolin-2-amine : 4, 4'-Bipyridine having a powder X-ray diffractogram displaying peaks expressed as degree 2-Theta angle at 12.0; 19.2; 21.2; and 24.3 (each time ±0.2), and which may optionally further show the following additional peaks expressed as degree 2-Theta angle: 16.0; 17.0; 17.8; 20.3; 22.5; and 22.7 (each time ±0.2); and even optionally further the following additional peaks expressed as degree 2-Theta angle: 8.5; 13.0; 15.7; 16.7; 20.9; 22.0; 23.1; 23.6 and 24.7 (each time ±0.2), optionally further characterized by a powder X-ray diffractogram and/or having a single endotherm with an onset temperature of 127.0°C (±2°C).

All the salt and crystalline form thereof may be obtained according to usual techniques known to the man skilled in the art.

According to particular embodiments, the compound or any one of its prodrugs or any one of its pharmaceutically acceptable salts, for use in a method for treating or preventing a *Coronaviridae* infection, is for reducing inflammation associated with the *Coronaviridae* infection.

According to particular embodiments, the compound or any one of its prodrugs or any one of its pharmaceutically acceptable salts, for use in a method for treating or preventing a *Coronaviridae* infection, is for reducing the *Coronaviridae* viral load.

According to particular embodiments, the compound or any one of its prodrugs or any one of its pharmaceutically acceptable salts, for use in a method for treating or preventing a *Coronaviridae* infection, is in combination with:
- a dynamin inhibitor as defined herein after, such as Dynasore; and/or
- an antibiotic, such as one selected from the group consisting of beta-lactams, fluoroquinolones, and macrolides; and/or
- an anti-inflammatory compound, such as one selected from the group consisting of: anti-TNF, Jak inhibitors, anti-IL6 antibodies, IL6 receptor antagonists.

According to some particular embodiments, the *Coronaviridae* is selected from *Letovirinae* and *Orthocoronavirinae.*

According to some particular embodiments, the *Coronaviridae* is an Alphacoronavirus or a Betacoronavirus or a Deltacoronavirus or a Gammacoronavirus.

According to some particular embodiments, the *Coronaviridae* is an Embecovirus or a Hibecovirus or a Merbecobivirus or a Nobecovirus or a Sarbecovirus.

According to some particular embodiments, the *Coronaviridae* is a Sarbecovirus selected from Severe Acute Respiratory Syndrome-related coronaviruses.

According to some particular embodiments, the Severe Acute Respiratory Syndrome (SARS)-related coronaviruses are selected from the group consisting of: SARS-CoV, SARSr-CoV WIV1, SARSr-CoV HKU3, SARSr-CoV RP3, SARS-CoV-2.

According to some preferred embodiments, the Severe Acute Respiratory Syndrome (SARS)-related coronaviruses are selected from SARS-CoV and SARS-CoV-2; including COVID-19 and their mutants.

According to some embodiments, the compounds of formula (I) or (II) or any one of their prodrugs or pharmaceutically acceptable salts are used in a method for treating or preventing a *Coronaviridae* infection, wherein the level of the compound, in a blood, plasma, tissue, saliva, pharyngeal, tracheal, bronchoalveolar, and/or serum sample of the patient, is measured during the use.

According to some of those embodiments, a presence and/or expression level of miR-124 is measured prior to and during the use.

### Dynamin inhibitors for use

As used herein, *"dynamin"* may refer to any polypeptide, natural or recombinant, which belongs to the *"dynamin superfamily",* including Dynamin I, Dynamin II and Dynamin III, in particular Dynamin II also referred herein as dynamin-2 and which is encoded in humans by the DNM2 gene.

As used herein a *"dynamin inhibitor"* may refer to any compound causing a decrease of the cellular content of the dynamin polypeptide, and/or the expression of the dynamin polypeptide, or the activity of the dynamin polypeptide, or the stability of the dynamin polypeptide. Non-limitative examples of inhibitors of dynamin expression encompass siRNAs, miRNAs, piRNAs that specifically bind to the dynamin-encoding nucleic acid or its corresponding mRNA, or alternatively, to a regulator of dynamin-expression. Examples of such inhibitors of dynamin expression encompass siRNAs, miRNAs, piRNAs that are complementary to such dynamin-encoding nucleic acid or its corresponding mRNA, or alternatively, to a regulator of dynamin-expression. Within the scope of the present invention, the term "complementary" is intended to mean that a first nucleic acid is complementary to a second nucleic acid when these nucleic acids have the base on each position which is the complementary (i.e. A to T, C to G) and in the reverse order. For example, the complementary sequence to TTAC is GTAA. If one strand of the double-stranded DNA is considered the sense strand, then the other strand, considered the antisense strand, will have the complementary sequence to the sense strand.

Within the scope of the present invention, the term *"dynamin stability",* such as in *"stability of the dynamin polypeptide"* or *"stability of the dynamin-encoding nucleic acid",* is intended to refer to the equilibrium reached between the synthesis and the degradation of the dynamin polypeptide or the dynamin-encoding nucleic acid.

Within the scope of the present invention *"an activator of dynamin activity"* is intended to refer to a compound able to increase, at least in part, the ability of the dynamin polypeptide to promote its physiological role in the cell; in particular its role in clathrin-mediated endocytosis.

Within the scope of the present invention *"an inhibitor of dynamin activity"* is intended to refer to a compound able to decrease, at least in part, the ability of the dynamin polypeptide to promote its physiological role in the cell; in particular its role in clathrin-mediated endocytosis.

According to one embodiment, the dynamin inhibitor may be selected from the group consisting of an antibody directed against Dynamin 2, or a nucleic acid molecule interfering specifically with Dynamin 2 expression.

According to one embodiment, the dynamin inhibitor may be selected from : 3-Hydroxynaphthalene-2-carboxylic acid (3,4-dihydroxybenzylidene)hydrazide, 3-Hydroxy-N'-[(2,4,5-trihydroxyphenyl)methylidene]naphthalene-2-carbohydrazide, tetradecyltrimethylammonium bromide, 4-Chloro-2-((2-(3-nitrophenyl)-1,3-dioxo- 2,3-dihydro-1H-isoindole-5-carbonyl)-amino)-benzoic acid, 2-Cyano-N-octyl-3-[1-(3-dimethylaminopropyl)-1H-indol-3 -yl] acrylamide, 3 -(2,4-Dichloro- -methoxyphenyl)- 2-sulfanylquinazolin-4(3H)-one, N,N'-(Propane-1,3-diyl)bis(7,8-dihydroxy-2-imino- 2H-chromene-3-carboxamide), N,N'-(Ethane- 1 ,2-diyl)bis(7,8-dihydroxy-2-imino-2H-chromene-3-carboxamide), OctadecylTriMethylAmmonium Bromide, Dynamin inhibitory peptide with aminoacid sequence: QVPS PNRAP, 3-Hydroxy-N'-[(2,4,5-trihydroxyphenyl)methylidene]naphthalene-2-carbohydrazide, and 4-(N,N-Dimethyl- N-octadecyl-N-ethyl)-4-aza-10-oxatricyclo-[5.2.1]decane-3,5-dione bromide.

In some embodiments, inhibitors of dynamin are inhibitors of receptor-mediated endocytosis which can be identified by methods that assay dynamin ring stabilization. These methods may comprise incubating a test agent with a dynamin polypeptide under conditions suitable for the formation of dynamin rings; and evaluating whether the test agent promotes accumulation of dynamin rings and/or inhibits disassembly of dynamin rings, the accumulation of dynamin rings and/or inhibition of disassembly of dynamin rings increasing basal dynamin GTPase activity. The evaluation of whether the test agent promotes the accumulation of dynamin rings or inhibits disassembly of dynamin rings can involve assaying for an increase in basal dynamin GTPase activity, and/or release of dynamin that is indicative of dynamin ring disassembly.

In some embodiments, dynamin-dependent endocytosis inhibitor is a dynamin GTPase inhibitor, illustrative examples of which are selected from compounds described U.S. Pat. Appl. Pub. No. 2007/0225363

Other representative compounds are selected from helical dynamin GTPase inhibitors, dimeric tyrphostins, dimeric benzylidenemalonitrile tyrphostins, iminochromenes, monomeric tyrphostins and 3-substituted naphthalene-2-carboxylic acid (benzylidene) hydrazides

According to a main embodiment, the invention relates to a dynamin inhibitor or any one of its or any one of its prodrugs or any one of its pharmaceutically acceptable salts, for use in a method for treating or preventing a *Coronaviridae* infection.

According to a particular embodiment, the dynamin inhibitor is a polypeptide.

In one framework of the present invention, the dynamin inhibitor is a dynamin inhibitor that targets the pleckstrin homology domain of dynamin.

As reported in Singh et al "dynamin functions and ligands: classical mechanism behind", Molecular pharmacology, 91:123-134, February 2017 various dynamin ligands have been reported such as dynasore, Napthohydrazide of formula (1) 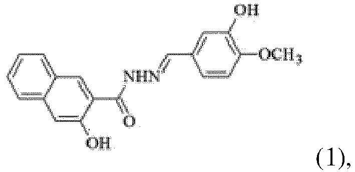 Napthoamide of formula (2) 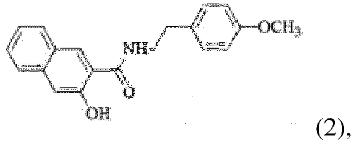 LRRK2IN1, 1,8-Nphthalimides of formula (3) 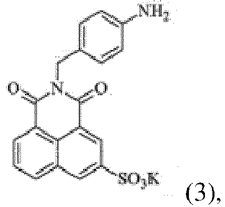 Pyrimdyn compound-6 of formula (4) 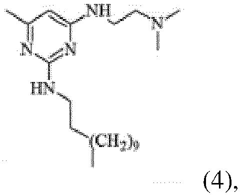 Rhodadyn A₁, Compound-5, DYRKla inhibitor of formula (5) 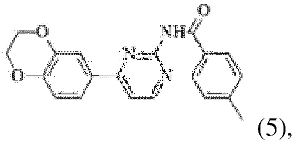 sertraline, indole-24 of formula (6) 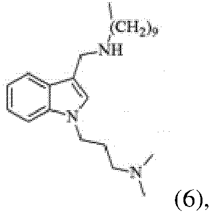 Phthaladyn-1 of formula (7) 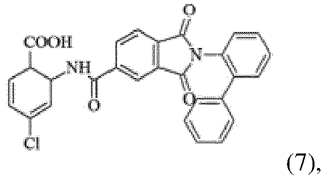 dynole-34 and dimethyl dynole of formula (8)

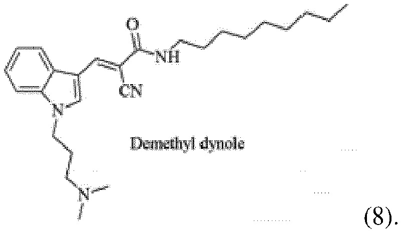

A further dynamin ligand may be cited having formula (9)

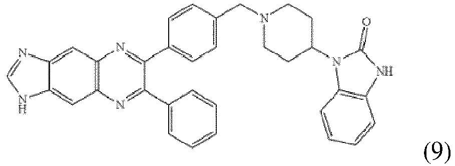

All of said dynamin ligands may be used in the framework of the present invention, alone or in combination, and as well as in combination with ABX464 or its N-glucuronide metabolite, as described above.

The chemical name for Dynasore is 3-Hydroxynaphthalene-2- carboxylic acid (3,4-dihydroxybenzylidene)hydrazide.

Among further dynamin inhibitor compounds, the following may be cited:
- Hydroxy-Dynasore which chemical name is: 3-Hydroxy-N'-[(2,4,5-trihydroxyphenyl)methylidene]naphthalene-2-carbohydrazide,
- Phthaladyn-23 which chemical name is 4-Chloro-2-((2-(3-nitrophenyl)-1,3-dioxo-2,3-dihydro-1H-isoindole-5-carbonyl)-amino)-benzoic acid,
- M-divi 1 which chemical name is 3-(2,4-Dichloro-5-methoxyphenyl)-2-sulfanylquinazolin-4(3H)-one,
- -Iminodyn-22/1 with the chemical name of Iminodyn 22 being N,N'-(Propane-1,3-diyl)bis(7,8-dihydroxy-2-imino-2H-chromene-3-carboxamide) and the chemical name of Iminodyn 17 being N,N'-(Ethane-1,2-diyl)bis(7,8-dihydroxy-2-imino-2H- chromene-3-carboxamide),
--Dyngo-4a which chemical name is 3-Hydroxy-N'-[(2,4,5-trihydroxyphenyl)methylidene]naphthalene-2-carbohydrazide, and
-RTIL-13 which chemical name is 4-(N,N-Dimethyl-N-octadecyl-N-ethyl)-4-aza-10-oxatricyclo- [5.2.1]decane-3,5-dione bromide.

In one embodiment, the dynamin inhibitor is a dynamin 2 inhibitor.

MiTMAB, OcTMAB, Dynasore and derivatives of Dynosore such as DD-6 or DD-11 are typical dynamin 2 inhibitors.

Long-chain acids, amines and ammonium salts are typical dynamin 1 inhibitors. 2-(dimethyl amino) ethyl myristate, tetradecylamin, DoTMAB, MiTMAB and OcTMAB may be cited.

According to one embodiment, the dynamin inhibitor is MiTMAB or Myristyl Trimethyl Ammonium Bromide, which is a dynamin 1 and 2 inhibitor of formula (10)

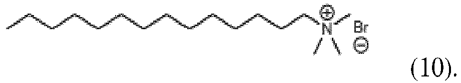

According to one embodiment, the dynamin inhibitor is DoTMAB of formula (11)

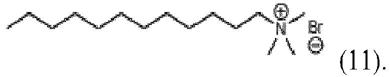

According to one embodiment, the dynamin inhibitor is OcTMAB or Octadecyl Trimethyl Ammonium Bromide of formula (12)

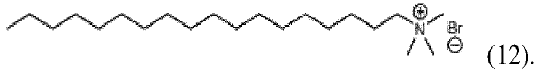

Further dynamin inhibitors may be cited such as an ammonium salt having formula (13) 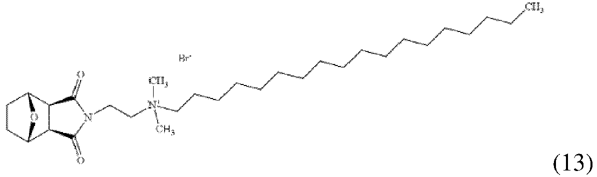 and compound having formula (14)

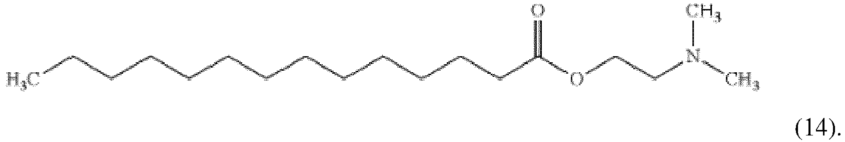

According to a further embodiment, the dynamin inhibitor is a phenothiazine derivative, well known in the pharmaceutical field.

Among such derivatives, the following may be cited chlorpromazine, promethazine and methylene blue, including 4-MB.

More generally, representative phenothiazine derivatives are:
- chlorpromazines such as acepromazine, chlorpromazine, cyamemazine, levomepromazine, oxomemazine, promazine, promethazine, triflupromazine,
- Pecazines, such as mesoridazine, metopimazine, pecazine, thioridazine,
- perphenazines, such as carfenazine, fluphenazine, perazine, perphenazine, prochlorperazine and trifluoperazine.

Another phenothiazine may be cited: methotrimeprazine.

Quinacrine and acridine may also be cited.

According to a particular embodiment, the dynamin inhibitor is selected from phenothiazine, Iminodyn-17, Iminodyn-22, Chlorpromazine, Dynasore, long chain amines and ammonium salts, such as MiTMABs and OcTMAB, dynoles, DD-6, desipramine, fluoxetine, reboxetine, fluphenazine, haloperidol and clozapine.

A dynamin inhibitor may comprise a mixture of dynamin inhibitors as described above.

According to a particular embodiment, the dynamin inhibitor for use in a method for treating or preventing a *Coronaviridae* infection, is a phenothiazine, or any one of its pharmaceutically acceptable salts.

According to a particular embodiment, the dynamin inhibitor for use in a method for treating or preventing a *Coronaviridae* infection, is a phenothiazine selected from the group consisting of: chlorpromazines, pecazines, and perphenazines, or any one of their pharmaceutically acceptable salts.

According to a particular embodiment, the dynamin inhibitor for use in a method for treating or preventing a *Coronaviridae* infection, is a chlorpromazine selected from the group consisting of: acepromazine, chlorpromazine, cyamemazine, levomepromazine, oxomemazine, promazine, promethazine, triflupromazine, or any one of their pharmaceutically acceptable salts.

According to a particular embodiment, the dynamin inhibitor for use in a method for treating or preventing a *Coronaviridae* infection, is a pecazine selected from the group consisting of: mesoridazine, metopimazine, pecazine, thioridazine, or any one of their pharmaceutically acceptable salts.

According to a particular embodiment, the dynamin inhibitor for use in a method for treating or preventing a *Coronaviridae* infection, is a perphenazine selected from the group consisting of: carfenazine, fluphenazine, perazine, perphenazine, prochlorperazine, trifluoperazine, or any one of their pharmaceutically acceptable salts.

According to a particular embodiment, the dynamin inhibitor for use in a method for treating or preventing a *Coronaviridae* infection, is a phenothiazine, Iminodyn-17, Iminodyn-22, Chlorpromazine, Dynasore, long chain amines, long chain ammonium salts, dynoles, DD-6, desipramine, fluoxetine, reboxetine, fluphenazine, haloperidol, clozapine, methylene blue, or any one of their pharmaceutically acceptable salts.

According to a particular embodiment, any one of the dynamin inhibitors reported herein for use in a method for treating or preventing a *Coronaviridae* infection, may be considered in combination with a compound of formula (I) or (II), or any one of their prodrugs or pharmaceutically acceptable salts; wherein compound (I) and compound (II) are respectively of formula (I) and (II)

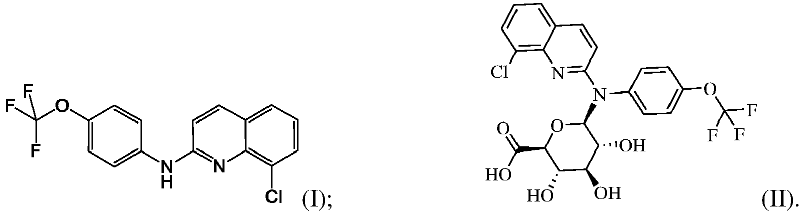

According to a particular embodiment, the dynamin inhibitor for use in a method for treating or preventing a *Coronaviridae* infection, is in combination with a cholesterol ester modulating agent.

According to a particular embodiment, the dynamin inhibitor for use in a method for treating or preventing a *Coronaviridae* infection, is in combination with chloroquine of hydrochloroquine.

According to a particular embodiment, the dynamin inhibitor for use in a method for treating or preventing a *Coronaviridae* infection, is in combination with at least one compound selected from (1) a compound of formula (I) or (II), or any one of their prodrugs or pharmaceutically acceptable salts; wherein compound (I) and compound (II) are respectively of formula (I) and (II) 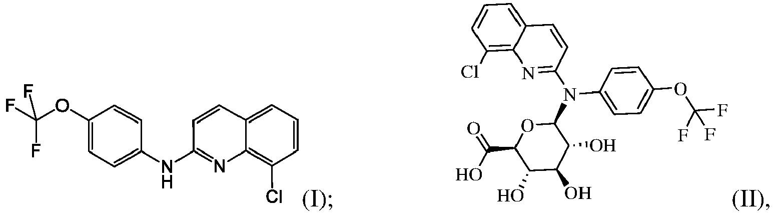 and/or (2) a cholesterol ester modulating agent and (3) chloroquine or hydrochloroquine.

### Pharmaceutical combinations with dynamin inhibitor

According to another main embodiment, the invention relates to a pharmaceutical composition comprising a dynamin inhibitor or one of its pharmaceutically acceptable salts; for use in a method for treating or preventing a *Coronaviridae* infection.

According to a particular embodiment of the invention, the dynamin inhibitor may be administered in combination with other compounds.

Thus, the pharmaceutical composition may further comprise at least one of compound (I) and (II), or any one of their prodrugs or pharmaceutically acceptable salts.

According to one embodiment, the dynamin inhibitor may be administered in combination with ABX464 or its N-glucuronide metabolite.

According to another embodiment, the dynamin inhibitor may be administered in combination with a cholesterol ester modulating agent, for example for increasing its stability.

Among such cholesterol ester modulating agent one may cite everolimus, pioglitazone, progesterone, verapamil and everolimus.

According to another embodiment, the dynamin inhibitor may be administered in combination with chloroquine or hydrochloroquine.

In the framework of the present invention, a combination may comprise a dynamin inhibitor in combination with at least one compound selected from (1) ABX464 or its N-glucuronide metabolite, (2) a cholesterol ester modulating agent and (3) chloroquine or hydrochloroquine.

Such combination may be suitable for separate administration, administration spread out over time or simultaneous administration to patients in need thereof.

The separate administration, simultaneous administration or administration spread out over time of a medicinal combination means that the elementary constituents of the combination, can be administered at the same time, each in one go at distinct moments, or repeatedly, or else at different moments, in particular during cycles. The elementary constituents can, in order to do this, be formulated as mixtures, only if they are administered simultaneously, or else formulated separately for the other administration schemes.

Triple combination comprising a dynamin inhibitor, ABX464 or its N-glucuronide metabolite and chloroquine or hydrochloroquine are thus encompassed within the scope of the present invention.

### Pharmaceutical compositions & Medicaments

According to a third main embodiment, the invention relates to a pharmaceutical composition comprising a compound of formula (I) or (II) as defined above or any one of its prodrugs or any one of its pharmaceutically acceptable salts, and at least one pharmaceutically acceptable excipient, for use in a method for treating or preventing a *Coronaviridae* infection.

In certain embodiments, a composition of this invention is formulated for administration to a patient in need of such composition. In some embodiments, a composition of this invention is formulated for oral administration or injectable, IV, IM, SC or sustained release or for inhalation to a patient.

According to a fourth main embodiment, the invention relates to a medicament comprising a compound of formula (I) or (II) as defined above, for use in a method for treating or preventing a *Coronaviridae* infection.

Alternatively, the invention relates to the use of a compound of formula (I) or (II) as defined above or any one of its prodrugs or any one of its pharmaceutically acceptable salts, for the preparation of a pharmaceutical composition or medicament for the treatment or prevention of a *Coronaviridae* infection.

Compositions of the present invention may be administered orally, parenterally, by inhalation aerosol, by spray, topically, rectally, nasally, buccally, vaginally, ophtalmologically or *via* an implanted reservoir. The term "parenteral" as used herein includes subcutaneous, intravenous, intramuscular, intra-articular, intra-synovial, intrasternal, intrathecal, intrahepatic, intralesional, intra-tracheal, and intracranial injection or infusion techniques. Preferably, the compositions are administered orally, intraperitoneally, intravenously or by inhalation. Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

For this purpose, any bland fixed oil may be employed including synthetic mono- or di-glycerides. Fatty acids, such as oleic acid and its glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

Pharmaceutically acceptable compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents may also be added.

Alternatively, pharmaceutically acceptable compositions of this invention may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols.

Pharmaceutically acceptable compositions of this invention may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation (see above) or in a suitable enema formulation. Topically transdermal patches may also be used.

For topical applications, provided pharmaceutically acceptable compositions may be formulated in a suitable ointment containing the active component suspended or dissolved in one or more carriers. Carriers for topical administration of compounds of this invention include, but are not limited to, mineral oil, liquid petrolatum, white petrolatum, propylene glycol, polyoxyethylene, polyoxypropylene compound, emulsifying wax and water. Alternatively, provided pharmaceutically acceptable compositions can be formulated in a suitable lotion or cream containing the active components suspended or dissolved in one or more pharmaceutically acceptable carriers.

Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, provided pharmaceutically acceptable compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutically acceptable compositions may be formulated in an ointment such as petrolatum.

According to a particular embodiment, pharmaceutically acceptable compositions of this invention may also be administered by nasal aerosol or inhalation.

Hence, according to a particular embodiment, the pharmaceutical composition is under a inhalation dosage form, a intraperitoneal dosage form or a intramuscular dosage form.

Hence, according to a particular embodiment, the pharmaceutical composition of the invention may be in the form of an intraperitoneal dosage form or a intramuscular dosage form.

Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents. Most preferably, pharmaceutically acceptable compositions of this invention are formulated for oral administration. Such formulations may be administered with or without food.

In some embodiments, pharmaceutically acceptable compositions of this invention are administered without food. In other embodiments, pharmaceutically acceptable compositions of this invention are administered with food.

The amount of compounds of the present invention that may be combined with the carrier materials to produce a composition in a single dosage form will vary depending upon the host treated, the particular mode of administration. Preferably, provided compositions should be formulated so that a dosage of between 0.01 - 100 mg/kg body weight/day of the inhibitor can be administered to a patient receiving these compositions.

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of a compound of the present invention in the composition will also depend upon the particular compound in the composition.

Such pharmaceutically acceptable compositions may also be considered in combination with other active compounds, or alternatively may include the compounds according to the invention in combination with other active agents.

In a non-limitative manner, such combinations with active agent(s) may thus consist of combinations with:
- a dynamin inhibitor as described above, such as Dynasore; and/or
- an antibiotic, such as one selected from the group consisting of beta-lactams, fluoroquinolones, and macrolides; and/or
- an anti-inflammatory compound, such as one selected from the group consisting of: anti-TNF, Jak inhibitors, anti-IL6 antibodies, IL6 receptor antagonists.

### Treatment monitoring and miR-124 as a biomarker & uses & methods thereof

In some embodiments, a method of the present invention for treating or preventing an infection of condition related to a virus, more particularly said virus having a RNA genome, and especially a RNA virus belonging to group IV according to the Baltimore classification, and more particularly for treating or preventing a *Coronaviridae* infection further comprises measuring a level of a compound or any one of its prodrugs or a pharmaceutically acceptable salt thereof as described herein, in a patient. In some embodiments, a level of a compound or any one of its prodrugs or a pharmaceutically acceptable salt thereof as described herein, is measured in a patient's biological sample. In some embodiments, a patient's biological sample is a blood, plasma, tissue, saliva, pharyngeal, tracheal, broncho alveolar and/or serum sample.

In a further embodiment, the invention provides the compound or any one of its prodrugs or any one of its pharmaceutically acceptable salts, for use in a method as defined above, wherein the level of a compound or any one of its prodrugs or a pharmaceutically acceptable salt thereof as described herein, in a blood, plasma, tissue, saliva, pharyngeal, tracheal, broncho alveolar and/or serum sample of the patient is measured during the use.

In some embodiments, a method of the present invention for treating an inflammatory disease, disorder or condition further comprises measuring a total level of compounds of formulas (I) and (II) as defined above, or pharmaceutically acceptable salts thereof, in a patient. In some embodiments, a method of the present invention for treating or preventing an infection of condition related to a virus, more particularly said virus having a RNA genome, and especially a RNA virus belonging to group IV according to the Baltimore classification, and more particularly for treating or preventing a *Coronaviridae* infection further comprises measuring a total level of compounds of formulas (I) and (II), or pharmaceutically acceptable salts thereof, in a patient.

In some embodiments, a method of the present invention for treating or preventing an infection of condition related to a virus, more particularly said virus having a RNA genome, and especially a RNA virus belonging to group IV according to the Baltimore classification, and more particularly for treating or preventing a *Coronaviridae* infection further comprises measuring and/or monitoring a presence and/or level of a biomarker in a patient. In some embodiments, a presence and/or level of a biomarker is measured in a patient's biological sample. In some embodiments, a patient's biological sample is a blood sample. In some embodiments, a patient's biological sample is a tissue sample. In some embodiments, a patient's biological sample is a pharyngeal, tracheal and/or broncho alveolar sample. In some embodiments, a biomarker measured and/or monitored in a method of the present invention is miR-124, as described in WO 2014/111892, the entire content of which is incorporated herein by reference. In some embodiments, a method of the present invention for treating or preventing an infection of condition related to a virus, more particularly said virus having a RNA genome, and especially a RNA virus belonging to group IV according to the Baltimore classification, and more particularly for treating or preventing a *Coronaviridae* infectionfurther comprises measuring and/or monitoring a presence and/or expression level of miR-124 in a patient prior to administering a compound or a pharmaceutically acceptable salt or composition thereof as described herein. In some embodiments, a method of the present invention for treating or preventing an infection of condition related to a virus, more particularly said virus having a RNA genome, and especially a RNA virus belonging to group IV according to the Baltimore classification, and more particularly for treating or preventing a *Coronaviridae* infectionfurther comprises measuring and/or monitoring a presence and/or expression level of miR-124 in a patient during the course of a treatment with a compound or a pharmaceutically acceptable salt or composition thereof as described herein. In some embodiments, a method of the present invention for treating or preventing an infection of condition related to a virus, more particularly said virus having a RNA genome, and especially a RNA virus belonging to group IV according to the Baltimore classification, and more particularly for treating or preventing a *Coronaviridae* infectionfurther comprises selecting a patient for a treatment with a compound or a pharmaceutically acceptable salt or composition thereof as described herein, by measuring and/or monitoring a presence and/or expression level of miR-124 in the patient. In some embodiments, a method of the present invention treating or preventing an infection of condition related to a virus, more particularly said virus having a RNA genome, and especially a RNA virus belonging to group IV according to the Baltimore classification, and more particularly for treating or preventing a *Coronaviridae* infection further comprises excluding a patient from a treatment with a compound or a pharmaceutically acceptable salt or composition thereof as described herein, by measuring and/or monitoring a presence and/or expression level of miR-124 in the patient. In some embodiments, a method of the present invention treating or preventing an infection of condition related to a virus, more particularly said virus having a RNA genome, and especially a RNA virus belonging to group IV according to the Baltimore classification, and more particularly for treating or preventing a *Coronaviridae* infection further comprises adjusting (such as increasing or decreasing) dosage regimen (such as dose amount and/or dose schedule) of a compound or a pharmaceutically acceptable salt or composition thereof as described herein to be administered to a patient, by measuring and/or monitoring a presence and/or expression level of miR-124 in the patient.

In some embodiments, a method of the present invention for treating or preventing an infection of condition related to a virus, more particularly said virus having a RNA genome, and especially a RNA virus belonging to group IV according to the Baltimore classification, and more particularly for treating or preventing a *Coronaviridae* infection comprises comparing a measured expression level of miR-124 in a patient to a control reference value. A control reference value to be used for comparing a measured expression level of miR-124 in a patient is obtained from a control sample. A control sample can be taken from various sources. In some embodiments, a control sample is taken from a patient prior to treatment or prior to the presence of a disease (such as an archival blood sample, pharyngeal, tracheal, broncho alveolar ortissue sample). In some embodiments, a control sample is taken from a set of normal, non-diseased members of a population. In some embodiments, a control sample is taken from a patient prior to treatment with a compound or a pharmaceutically acceptable salt or composition thereof as described herein. In some embodiments, a cell assay can be performed on a biological sample.

In some embodiments, a modulated presence and/or expression level of miR-124 in a patient compared to a control reference value indicates an infection of condition related to a virus, more particularly said virus having a RNA genome, and especially a RNA virus belonging to group IV according to the Baltimore classification, and more particularly a *Coronaviridae* infection. In some embodiments, a modulated presence and/or expression level of miR-124 in a patient compared to a control reference value indicates an efficacy of a treatment with a compound or a pharmaceutically acceptable salt or composition thereof as described herein, which is administered to the patient. The terms "modulation" or "modulated presence and/or expression level" means the presence or expression level of a biomarker is either induced or increased, or alternatively is suppressed or decreased.

In some embodiments, a measured reduced or suppressed presence, or a decreased expression level, of miR-124 relative to a control reference value indicates an infection of condition related to a virus, more particularly said virus having a RNA genome, and especially a RNA virus belonging to group IV according to the Baltimore classification, and more particularly for treating or preventing a *Coronaviridae* infection. In some embodiments, a measured induced or increased presence, or an increased expression level, of miR-124 relative to a control reference value indicates an efficacy of a compound or a pharmaceutically acceptable salt or composition thereof as described herein. In some embodiments, a measured expression level of miR-124 in a patient treated with a compound or a pharmaceutically acceptable salt or composition thereof as described herein is a two-fold, four-fold, six-fold, eight-fold, or ten-fold increase relative to a control reference value.

Thus, according to a particular embodiment, the present invention further provides the compound or any one of its prodrugs or any one of its pharmaceutically acceptable salts, for use in a method for treating or preventing a *Coronaviridae* infection, as defined above, wherein a presence and/or expression level of miR-124 in a blood and/or tissue sample of the patient is measured prior to and during the use.

According to a fifth main embodiment, the invention relates to an *in vitro* or *ex vivo* use of at least one miRNA, said at least one miRNA being miR-124, as a biomarker of a *Coronaviridae* infection, or of an efficacy of a therapeutic treatment of said *Coronaviridae* infection.

According to a sixth main embodiment, the invention relates to an *in vitro* or *ex vivo* method for assessing a *Coronaviridae* infection in a patient presumed to be infected with a virus, comprising at least the steps of:
a- measuring a presence or an expression level of at least one miRNA, said at least one miRNA being miR-124, in a biological sample previously obtained from said patient; and
b- comparing said presence or expression level to a control reference value, wherein a modulated presence or level of expression of said miRNA relative to said control reference value is indicative of a *Coronaviridae* infection.

According to one embodiment, uses and methods according to the invention may, in particular, allow for the determining of a *Coronaviridae* infection in a patient, and in particular for the follow-up of such infection.

According to one embodiment, a presence or a level of expression of miR-124 is measured into an isolated biological sample, and then is compared to a control reference value.

A modulation of the presence or level of expression of miR-124 relative to the control reference value may be indicative of a viral infection. In particular a reduced or suppressed presence, or a decreased level of expression, of said miRNA relative to a control reference value may be indicative of a viral infection.

In one embodiment, a use of the invention may comprise obtaining of a measured level of expression of said miR-124 into an isolated biological sample and comparing said measured level of expression to a control reference value. An observation of a modulation of said measured level relative to said control reference value may be indicative of a viral infection, or of an efficacy of a therapeutic treatment of said viral infection.

When miR-124 from a sample is "decreased" or "down-regulated" in a biological sample isolated from a patient, as compared to a control reference value, this decrease can be, for example, of about 5%, 10%, 20%, 30%, 40%, 50%, 60%, 70%, 90%, 100%, 200%, 300%, 500%, 1,000%, 5,000% or more of the comparative control reference value (i.e., without the treatment by the quinoline derivative).

In particular, the measured level expression of miR-124 may be at least a two-fold, preferably at least a four-fold, preferably at least a six-fold, preferably at least an eight-fold, and more preferably at least a ten-fold decrease relative to said control reference value.

According to one embodiment, uses of and methods implementing miR-124 as a biomarker for a *Coronaviridae* infection, may be combined with the determination of others biomarkers specific from said infection such as the determination of the presence or level of expression of peptides, proteins or nucleic acid sequences specific from said virus.

According to one embodiment, the increase of the presence or level of expression of miR-124 in a biological sample taken from a patient suffering from a *Coronaviridae* infection and receiving a treatment for this infection relative to a biological sample taken from the same patient before initiating said treatment may be indicative of the severity of the disease or efficacy of said treatment.

According to one embodiment, the uses and methods of the invention may be for assessing a responsiveness of a patient to a treatment with said compounds of formula (I) or (II).

According to another embodiment, the uses and methods of the invention may be for assessing an effectiveness of a treatment with said compounds of formula (I) or (II).

According to another embodiment, the uses and methods of the invention may be for assessing a therapeutic efficacy of said compounds of formula (I) or (II) as a therapeutic agent for preventing and/or treating a *Coronaviridae* infection.

According to one embodiment, the uses and methods of the invention may be for assessing a patient compliance with a treatment with said compounds of formula (I) or (II).

The miR-124 biomarker may be used to monitor or manage compounds of formula (I) or (II) activity during patient treatment of a *Coronaviridae* infection

According to one embodiment, a use or a method according to the invention may be implemented for optimizing the dosing regimen of a patient. Patients may respond differently to a given compound of formula (I) or (II), depending on such factors as age, health, genetic background, presence of other complications, disease progression, and the co-administration of other drugs. It may be useful to utilize the miR-124 biomarker to assess and optimize the dosage regimen, such as the dose amount and/or the dose schedule, of a quinoline derivative in a patient. In this regard, miR-124-based biomarker can also be used to track and adjust individual patient treatment effectiveness over time. The biomarker can be used to gather information needed to make adjustments in a patient's treatment, increasing or decreasing the dose of an agent as needed. For example, a patient receiving a compound of formula (I or (II) can be tested using the miR-124 -based biomarker to see if the dosage is becoming effective, or if a more aggressive treatment plan needs to be put into place. The amount of administered drug, the timing of administration, the administration frequency, the duration of the administration may be then adjusted depending on the miR-124 biomarker measurement.

The miR-124 biomarker may also be used to track patient compliance during individual treatment regimes, or during clinical trials. This can be followed at set intervals to ensure that the patients included in the trial are taking the drugs as instructed. Furthermore, a patient receiving a quinoline derivative can be tested using the miR-124 biomarker to determine whether the patient complies with the dosing regimen of the treatment plan. An increased expression level of the biomarker compared to that of an untreated control sample is indicative of compliance with the protocol.

A biomarker of the invention may be implemented to assess and follow the efficacy of compounds of formula (I) or (II). Accordingly, a presence or level of expression of miR-124 may be measured into an isolated biological sample obtained from a patient previously treated with compounds of formula (I) or (II). Then, the measured presence or level expression of miR-124 into an isolated biological sample may be compared to a control reference value.

When an increase of the measured level relative to the control reference value is observed, then the measure is indicative of an activity of said compounds of formula (I) or (II).

In another embodiment, when an increase of the measured level relative to the control reference value is observed, then the measure may be indicative of a responsiveness of a patient to a treatment with said compounds of formula (I) or (II).

In another embodiment, when an increase of the measured level relative to the control reference value is observed, then the measure may be indicative of an effectiveness of a treatment with said compounds of formula (I) or (II).

In another embodiment, when an increase of the measured level of expression relative to the control reference value is observed, then the measure may be indicative a therapeutic efficacy of said compounds of formula (I) or (II) as a therapeutic agent for preventing and/or treating a *Coronaviridae* infection.

When miR-124 from a sample is "increased" or "up-regulated" after a treatment with a quinoline derivative, as compared to a non-treated control reference value, this increase can be, for example, of about 5%, 10%, 20%, 30%, 40%, 50%, 60%, 70%, 90%, 100%, 200%, 300%, 500%, 1,000%, 5,000% or more of the comparative control reference value (i.e., without the treatment by the compounds of formula (I) or (II).

In particular, the measured level expression of miR-124 may be at least a two-fold, preferably at least a four-fold, preferably at least a six-fold, preferably at least an eight-fold, and more preferably at least a ten-fold increase relative to said control reference value.

According to another embodiment of the invention, when monitoring a *Coronaviridae* infection or assessing an efficacy of a *Coronaviridae* infection treatment, in particular with a compound of formula (I) or (II), a patient may be tested with a method or a use of the invention at a time interval selected from the group consisting of hourly, twice a day, daily, twice a week, weekly, twice a month, monthly, twice a year, yearly, and every other year. The then collected sample can be tested immediately, or can be stored for later testing.

According to another embodiment, use and methods according to the invention may, in particular, allow for the screening, identification or evaluation of potential active agents as a drug candidate.

In particular, use and methods according to the invention are particularly advantageous for the screening, identification or evaluation of potential active agents, such as a drug candidate or a vaccine presumed effective towards a *Coronaviridae* infection.

According to another embodiment of the invention, a miR-124 biomarker may be implemented to screen a drug candidate or a vaccine candidate presumed effective for preventing and/or treating a *Coronaviridae* infection. In such embodiment, a presence or level of expression of miR-124 may be measured into an isolated biological sample or isolated cell previously contacted with the drug or vaccine to be screened. Then, the obtained measure may be compared to a control reference value.

When an increase of the measured level into an isolated biological sample or isolated cell, previously contacted with the compound, drug or vaccine candidate to be screened, relative to a control reference value is observed, then the measure may be indicative of said candidate to have a biological effect and in particular to be efficient for altering the physiological activity of a cell.

In particular, a drug candidate or vaccine candidate may be characterized as being efficient in preventing and/or treating the *Coronaviridae* infection

When miR-124 from a sample is "increased" or "up-regulated" after treatment with a drug candidate, as compared to a non- treated control reference value, this increase can be, for example, of about 5%, 10%, 20%, 30%, 40%, 50%, 60%, 70%, 90%, 100%, 200%, 300%, 500%, 1,000%, 5,000% or more of the comparative control reference value i.e., without the treatment by the compound of formula (I) or (II).

In particular, the measured level expression of miR-124 may be at least a two-fold, preferably at least a four-fold, preferably at least a six-fold, preferably at least an eight-fold, and more preferably at least a ten-fold increase relative to said control reference value.

The uses and methods of the invention may comprise measuring a level of expression of miR-124 into an isolated biological sample. Any suitable sample may be used to assess the miR-124 biomarker.

The step of collecting biological samples for the uses and methods of the invention is performed before carrying out the invention and is not a step of a use or a method in accordance with the invention.

Samples for miRNA assessment can be taken during any desired intervals. For example, samples can be taken hourly, twice per day, daily, weekly, monthly, every other month, yearly, or the like. The sample can be tested immediately, or can be stored for later testing.

The samples can be purified prior to testing. In some embodiments, the miR-124 can be isolated from the remaining cell contents prior to testing. Further, the miR-124 molecules can be separated from the rest of the mRNA in the sample, if desired. For example, the miR-124 can be separated from the mRNA based on size differences prior to testing.

Control reference value to be used for comparing the measured level of expression of miR-124 in a tested biological sample is obtained from a control sample.

Control samples can be taken from various sources. In some embodiments, control samples are taken from the patient prior to treatment or prior to the presence of the disease (such as an archival blood sample). In other embodiments, the control samples are taken from a set of normal, non-diseased members of a population. In another embodiment, a cell assay can be performed on a control cell culture, for example, that has not been treated with the test compound or has been treated with a reference compound, such as the 8-chloro-N-[4-(trifluoromethoxy)phenyl]quinolin-2-amine.

According to one embodiment, for the determination or monitoring of a viral infection in a patient, a control reference value may be obtained from an isolated biological sample obtained on an individual or group of individuals known to not suffer from such condition.

According to another embodiment, for the determination or monitoring of an efficacy of a treatment of a viral infection into a patient, a control reference value may be obtained from an isolated biological sample obtained from an individual or group of individuals known to not suffer from such condition, and not receiving the treatment the efficacy of which is to be determined or monitored. Alternatively, a control reference value may be obtained from an isolated biological sample obtained from a patient suffering from a viral infection and receiving a treatment the efficacy of which being to be determined or monitored, the isolated biological sample being taken from the patient before administration of the treatment.

Numerous methods are available to the skilled man to measure a presence or level of expression of the miR-124 biomarker.

For example, nucleic acid assays or arrays can be used to assess the presence and/or expression level of miR-124 in a sample.

The sequence of the miR-124 may be used to prepare a corresponding nucleotide acting as complementary probe or primer to be used in different nucleic acid assays for detecting the expression or presence of the miR-124 biomarker in the sample, such as, but not limited to, Northern blots and PCR-based methods (e.g., Real-Time Reverse Transcription-PCR or qRT-PCR). Methods such as qRT-PCR may be used to accurately quantitate the amount of the miRNA in a sample.

Sense and anti-sense probes or primers according to the invention may be obtained using every process known to the man skilled in the art, in particular those that are described in Sambrook et al. (Molecular Cloning: A Laboratory Manual, 3rd ED., 2001, Cold Spring Harbour, N. Y.).

Methods related to the detection and quantification of RNA or DNA are well known in the art. The man skilled in the art may for instance refer to Wang et al. (1989, Proc Natl Acad Sci USA, Vol. 86 : 917-921), de Wong et al. (2005, Bio Techniques, Vol. 39 (1): 75-85), de Nolan et al. (2006, Nat Protoc, Vol. 1(3) : 1559-1582) et de Klinck et al. (2008, Cancer Research, Vol. 68 : 657-663), or also to a general review published by Bustin (2000, Journal of Molecular Endocrinology, Vol. 25 : 169-193).

In one embodiment, a method for the detection and quantification of nucleic acids may be a fluorescent-dye-based method, wherein nucleic acid concentration is assessed by measuring the fluorescence intensity of ligands, such as dyes, that bind to said nucleic acids. Fluorescent dyes are well known in the art.

Alternatively, said nucleic acid may be quantified using spectrophotometry.

In another embodiment, a method for the detection and quantification of nucleic acids may be a hybridation-based method. Said hybridation-based methods may include PCR and quantitative-PCR (qRT-PCR or q-PCR) techniques or reverse transcriptase / polymerase based techniques. Advantageously, said method may comprise, or be further combined, with a sequencing step.

Those methods may comprise (i) a step of extraction of cellular mRNAs, (ii) a step of reverse transcription of mRNA to DNA using a reverse transcriptase and (iii) a step of DNA amplification from DNA obtained on the previous step. Usually, starting from the same sample, the following nucleic acids are amplified : (a) DNA obtained after a reverse transcription step of the target mRNA and (b) a DNA or a plurality of DNAs obtained after reverse transcription of mRNAs which are constitutively and constantly expressed by cells (« housekeeping genes »), such as RNAs coded by genes *MRPL19, PUM1* and *GADPH.*

The amplified DNA can be quantified, after separation by electrophoresis, and measure of DNA bands. Results related to the target mRNA(s) are expressed as relative units in comparison to mRNAs coded by « housekeeping » genes. In some embodiments, the step of separation of amplified DNAs is achieved after agarose gel electrophoresis, and then coloration of DNA bands with ethidium bromide, before quantification of DNA contained in those migration bands with densitometry. In other embodiments, one may use a micro-channel device in which amplified DNA is separated by capillar electrophoresis, before quantification of the emitted signal using a laser beam. Such a device may be a LabChip® device, for instance from the « GX » series, commercialized by the company Caliper LifeSciences (Hopkinton, MA, USA).

Quantitative results obtained by qRT-PCR can sometimes be more informative than qualitative data, and can simplify assay standardization and quality management. Thus, in some embodiments, qRT-PCR-based assays can be useful to measure miRNA levels during cell-based assays. The qRT-PCR method may be also useful in monitoring patient therapy. Commercially available qRT-PCR based methods {e.g., TaqmanR Array™)

Any suitable assay platform can be used to determine the expression or presence of the miRNA in a sample. For example, an assay may be in the form of a dipstick, a membrane, a chip, a disk, a test strip, a filter, a microsphere, a slide, a multiwell plate, or an optical fiber. An assay system may have a solid support on which an oligonucleotide corresponding to the miRNA is attached. The solid support may comprise, for example, a plastic, silicon, a metal, a resin, glass, a membrane, a particle, a precipitate, a gel, a polymer, a sheet, a sphere, a polysaccharide, a capillary, a film a plate, or a slide. The assay components can be prepared and packaged together as a kit for detecting an miRNA.

In some embodiments, an oligonucleotide array for testing for the compound or drug candidate activity in a biological sample can be prepared or purchased. An array typically contains a solid support and at least one oligonucleotide contacting the support, where the oligonucleotide corresponds to at least a portion of the miR-124 biomarker. In some embodiments, the portion of the miR-124 biomarker comprises at least 5, 10, 15, 20 or more bases.

According to one embodiment, the presence or expression of miR-124 may be assayed in combination with others miRNA also used as biomarkers. In such an embodiment, an array can be used to assess the expression or presence of multiple miRNAs in a sample, including miRNA-124. In general, the method comprises the following steps: a) contacting the sample with an array comprising a probe set under conditions sufficient for specific binding to occur; and b) examining the array to detect the presence of any detectable label, thereby evaluating the amount of the respective target miRNAs in the sample. The use of an expression array allows obtaining a miRNA expression profile for a given sample.

Methods of preparing assays or arrays for assaying miRNAs are well known in the art and are not needed to be further detailed here.

Nucleic acid arrays can be used to detect presence or differential expression of miRNAs in biological samples. Polynucleotide arrays (such as DNA or RNA arrays) typically include regions of usually different sequence polynucleotides ("capture agents") arranged in a predetermined configuration on a support. The arrays are "addressable" in that these regions (sometimes referenced as "array features") have different predetermined locations ("addresses") on the support of array. The region (i.e., a "feature" or "spot" of the array) at a particular predetermined location (i.e., an "address") on the array will detect a particular miRNA target. The polynucleotide arrays typically are fabricated on planar supports either by depositing previously obtained polynucleotides onto the support in a site specific fashion or by site specific in situ synthesis of the polynucleotides upon the support. Arrays to detect miRNA expression can be fabricated by depositing (e.g., by contact- or jet-based methods or photolithography) either precursor units (such as nucleotide or amino acid monomers) or pre-synthesized capture agent. After depositing the polynucleotide capture agents onto the support, the support is typically processed (e.g., washed and blocked for example) and stored prior to use.

An array to detect miRNA expression has at least two, three, four, or five different subject probes. However, in certain embodiments, a subject array may include a probe set having at least 10, at least 20, at least 50, at least 100, at least 200, at least 500, or at least 1,000 or more probes that can detect a corresponding number of miRNAs. In some embodiments, the subject arrays may include probes for detecting at least a portion or all of the identified miRNAs of an organism, or may include orthologous probes from multiple organisms.

A nucleic acid array may be contacted with a sample or labeled sample containing miRNA analytes under conditions that promote specific binding of the miRNA in the sample to one or more of the capture agents present on the array to exhibit an observed binding pattern. This binding pattern can be detected upon interrogating the array. For example, the target miRNAs in the sample can be labeled with a suitable label (such as a fluorescent compound), and the label then can be accurately observed (such as by observing the fluorescence pattern) on the array after exposure of the array to the sample. The observed binding pattern can be indicative of the presence and/or concentration of one or more miRNA components of the sample.

The labeling of miRNAs may be carried using methods well known in the art, such as using DNA ligase, terminal transferase, or by labeling the RNA backbone, etc. In some embodiments, the miRNAs may be labeled with fluorescent label. Exemplary fluorescent dyes include but are not limited to xanthene dyes, fluorescein dyes, rhodamine dyes, fluorescein isothiocyanate (FITC), 6 carboxyfluorescein (FAM), 6 carboxy-2 1 ,4 1 ,7',4,7-hexachlorofluorescein (HEX), 6 carboxy 4', 5' dichloro 2', 7' dimethoxyfluorescein (JOE or J), N,N,N',N' tetramethyl 6 carboxyrhodamine (TAMRA or T), 6 carboxy X rhodamine (ROX or R), 5 carboxyrhodamine 6G (R6G5 or G5), 6 carboxyrhodamine 6G (R6G6 or G6), and rhodamine 110; cyanine dyes, e.g. Cy3, Cy5 and Cy7 dyes; Alexa dyes, e.g. Alexa-fluor-555; coumarin, Diethylaminocoumarin, umbelliferone; benzimide dyes, e.g. Hoechst 33258; phenanthridine dyes, e.g. Texas Red; ethidium dyes; acridine dyes; carbazole dyes; phenoxazine dyes; porphyrin dyes; polymethine dyes, BODIPY dyes, quinoline dyes, Pyrene, Fluorescein Chlorotriazinyl, R1 10, Eosin, JOE, R6G, Tetramethylrhodamine, Lissamine, ROX, Naptho fluorescein, and the like.

In some embodiments, an oligonucleotide array for assessing immunomodulatory activity can be prepared or purchased, for example from Affymetrix. The array may contain a solid support and a plurality of oligonucleotides contacting the support. The oligonucleotides may be present in specific, addressable locations on the solid support; each corresponding to at least a portion of miRNA sequences which may be differentially expressed upon treatment of a quinoline derivative or a drug candidate in a cell or a patient. The miRNA sequences comprise at least one miR-124 sequence.

When an array is used to assess miRNAs, a typical method can contain the steps of 1) obtaining the array containing surface-bound subject probes; 2) hybridization of a population of miRNAs to the surface-bound probes under conditions sufficient to provide for specific binding (3) post-hybridization washes to remove nucleic acids not bound in the hybridization; and (4) detection of the hybridized miRNAs. The reagents used in each of these steps and their conditions for use may vary depending on the particular application.

Hybridization can be carried out under suitable hybridization conditions, which may vary in stringency as desired. Typical conditions are sufficient to produce probe/target complexes on an array surface between complementary binding members, i.e., between surface-bound subject probes and complementary miRNAs in a sample. In certain embodiments, stringent hybridization conditions may be employed. Hybridization is typically performed under stringent hybridization conditions. Standard hybridization techniques which are well-known in the art (e.g. under conditions sufficient to provide for specific binding of target miRNAs in the sample to the probes on the array) are used to hybridize a sample to a nucleic acid array. Selection of appropriate conditions, including temperature, salt concentration, polynucleotide concentration, hybridization time, stringency of washing conditions, and the like will depend on experimental design, including source of sample, identity of capture agents, degree of complementarity expected, etc., and may be determined as a matter of routine experimentation for those of ordinary skill in the art. In general, a "stringent hybridization" and "stringent hybridization wash conditions" in the context of nucleic acid hybridization are typically sequence dependent, and are different under different experimental conditions. Hybridization may be done over a period of about 12 to about 24 hours. The stringency of the wash conditions can affect the degree to which miRNA sequences are specifically hybridized to complementary capture agents. Those of ordinary skill will readily recognize that alternative but comparable hybridization and wash conditions can be utilized to provide conditions of similar stringency.

As an illustration, in one embodiment, the miRNA expression profiling experiments may be conducted using the Affymetrix Genechip miRNA Array 2.0 and following the protocols described in the instruction manual.

In one particular embodiment, said hybridization can be performed using the GeneChip® Hybridization, Wash, and Stain Kit (Affymetrix Ref. #900720). Advantageously, said hybridization is performed by following the protocols of the manufacturer.

After the miRNA hybridization procedure, the array-surface bound polynucleotides are typically washed to remove unbound nucleic acids. Washing may be performed using any convenient washing protocol, where the washing conditions are typically stringent, as described above. For instance, a washing step may be performed using washing buffers sold by the company Affymetrix (Ref. #900721 and #900722). The hybridization of the target miRNAs to the probes is then detected using standard techniques of reading the array. Reading the resultant hybridized array may be accomplished, for example, by illuminating the array and reading the location and intensity of resulting fluorescence at each feature of the array to detect miRNA/probe binding complexes.

## Claims

1. Compound of formula (I) 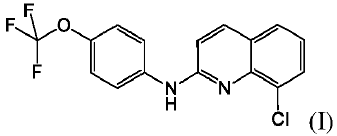 or any one of its prodrugs or any one of its pharmaceutically acceptable salts, for use in a method for treating or preventing a *Coronaviridae* infection.

2. Compound of formula (II) 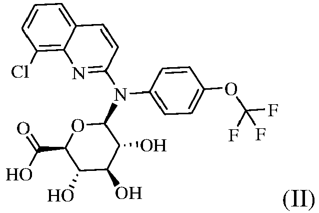 or any one of its prodrugs or any one of its pharmaceutically acceptable salts for use in a method for treating or preventing a *Coronaviridae* infection.

3. The compound or any one of its prodrugs or any one of its pharmaceutically acceptable salts, for use in a method for treating or preventing a *Coronaviridae* infection, according to claim 1 or claim 2, wherein the pharmaceutically acceptable salts are selected from the group consisting of:
- salts formed with inorganic acids such as hydrochloric acid, hydrobromic acid, phosphoric acid, sulfuric acid, persulfuric acid, boric acid and perchloric acid,
- salts formed with organic acids such as acetic acid, oxalic acid, maleic acid, tartaric acid, citric acid, succinic acid or malonic acid, and
- one salt selected from adipate, alginate, ascorbate, aspartate, benzoate, edetate, gluceptate, bisulfate, borate, butyrate, camphorate, cyclopentaneproprionate, citrate, glycerophosphoric acid, nitric acid, cyclopentanepropionate, digluconate, dodecylsulfate, formate, acetate, fumarate, glucoheptonate, glycerophosphate, gluconate, hemisulfate, glucoheptonate, heptanoate, hexanoate, hydroiodide, lactobionate, lactate, laurate, lauryl sulfate, malate, maleate, malonate, mandelate, salicylate, disalicylate, picrate, mucate, , nicotinate, nitrate, oleate, oxalate, palmitate, pamoate, pectinate, persulfate, dodecylsulfate, 3-phenylpropionate, phosphate, pivalate, propionate, undecanoate stearate, succinate, bitartrate, sulfate, tartrate, trifluoroacetate, triflate, thiocyanate, undecanoate, valerate salts, pantothenate, dodecylsulfate, sulfonate, in particular alkylsufonate such as methanesulfonate (or mesylate), esylate, edisylate, estolate, ethanesulfonate, 2- hydroxy-ethanesulfonate or arylsulfonate, such as 2-naphthalenesulfonate, napadisylate, napsylate, camphorsulfonate, benzenesulfonate and p-toluenesulfonate.

4. The compound or any one of its prodrugs or any one of its pharmaceutically acceptable salts, for use in a method for treating or preventing a *Coronaviridae* infection, according to claim 3, wherein the salt is selected from sulfate, hydrobromide, citrate, trifluoroacetate, ascorbate, hydrochloride, tartrate, triflate, maleate, mesylate, formate, acetate, fumarate and sulfonate, in particular alkylsufonate or arylsulfonate, and more particularly mesylate, triflate, edisylate, besylate and tosylate.

5. The compound or any one of its prodrugs or any one of its pharmaceutically acceptable salts, for use in a method for treating or preventing a *Coronaviridae* infection, according to anyone of claims 1 to 4, wherein the *Coronaviridae* is selected from *Letovirinae* and *Orthocoronavirinae.*

6. The compound or any one of its prodrugs or any one of its pharmaceutically acceptable salts, for use in a method for treating or preventing a *Coronaviridae* infection, according to claim 5, wherein the *Coronaviridae* is an Alphacoronavirus or a Betacoronavirus or a Deltacoronavirus or a Gammacoronavirus.

7. The compound or any one of its prodrugs or any one of its pharmaceutically acceptable salts, for use in a method for treating or preventing a *Coronaviridae* infection according to claim 6, wherein the *Coronaviridae* is an Embecovirus or a Hibecovirus or a Merbecobivirus or a Nobecovirus or a Sarbecovirus.

8. The compound or any one of its prodrugs or any one of its pharmaceutically acceptable salts, for use in a method for treating or preventing a *Coronaviridae* infection according to claim 7, wherein the *Coronaviridae* is a Sarbecovirus selected from Severe Acute Respiratory Syndrome-related coronaviruses.

9. The compound or any one of its prodrugs or any one of its pharmaceutically acceptable salts, for use in a method for treating or preventing a *Coronaviridae* infection according to claim 8, wherein the Severe Acute Respiratory Syndrome (SARS)-related coronaviruses are selected from the group consisting of: SARS-CoV, SARSr-CoV WIV1, SARSr-CoV HKU3, SARSr-CoV RP3, SARS-CoV-2; including COVID-19 and their mutants.

10. The compound or any one of its prodrugs or any one of its pharmaceutically acceptable salts, for use in a method for treating or preventing a *Coronaviridae* infection, according to claim 9, wherein the Severe Acute Respiratory Syndrome (SARS)-related coronaviruses are selected from SARS-CoV and SARS-CoV-2.

11. The compound or any one of its prodrugs or any one of its pharmaceutically acceptable salts, for use in a method for treating or preventing a *Coronaviridae* infection, according to anyone of claims 1 to 10, wherein the level of the compound, in a blood, plasma, tissue, saliva, pharyngeal, tracheal, bronchoalveolar, and/or serum sample of the patient, is measured during the use.

12. The compound or any one of its prodrugs or any one of its pharmaceutically acceptable salts, for use in a method for treating or preventing a *Coronaviridae* infection, according to anyone of claims 1 to 11, wherein a presence and/or expression level of miR-124 in a blood and/or tissue sample of the patient is measured prior to and during the use.

13. Pharmaceutical composition comprising a compound or any one of its prodrugs or any one of its pharmaceutically acceptable salts as defined in anyone of claims 1 to 12, and at least one pharmaceutically acceptable excipient, for use in a method for treating or preventing a *Coronaviridae* infection.

14. The pharmaceutical composition according to claim 13, wherein the pharmaceutical composition is under a inhalation dosage form, a intraperitoneal dosage form or a intramuscular dosage form.

15. Medicament comprising a compound as defined in any one of claims 1 to 12, for use in a method for treating or preventing a *Coronaviridae* infection.

16. An *in vitro* or *ex vivo* use of at least one miRNA, said at least one miRNA being miR-124, as a biomarker of a *Coronaviridae* infection, or of an efficacy of a therapeutic treatment of said *Coronaviridae* infection.

17. The use according to claim 16, wherein a measured level of expression of said miR-124 into an isolated biological sample is compared to a control reference value, and wherein a modulation of said measured level relative to said control reference value is indicative of a *Coronaviridae* infection, or of an efficacy of a therapeutic treatment of said *Coronaviridae* infection.

18. The use according to claim 17, wherein said biological sample is selected in a group consisting of a biological tissue sample, a whole blood sample, a swab sample, a plasma sample, a serum sample, a saliva sample, a vaginal fluid sample, a sperm sample, a pharyngeal fluid sample, a bronchial fluid sample, a fecal fluid sample, a cerebrospinal fluid sample, a lacrymal fluid sample and a tissue culture supernatant sample.

19. An *in vitro* or *ex vivo* method for assessing a *Coronaviridae* infection in a patient presumed to be infected with a virus, comprising at least the steps of:
a- measuring a presence or an expression level of at least one miRNA, said at least one miRNA being miR-124, in a biological sample previously obtained from said patient; and
b- comparing said presence or expression level to a control reference value,
wherein a modulated presence or level of expression of said miRNA relative to said control reference value is indicative of a *Coronaviridae* infection.

20. The compound or any one of its prodrugs or any one of its pharmaceutically acceptable salts, for use in a method for treating or preventing a *Coronaviridae* infection, according to anyone of claims 1 to 12; which is for reducing inflammation associated with the *Coronaviridae* infection.

21. The compound or any one of its prodrugs or any one of its pharmaceutically acceptable salts, for use in a method for treating or preventing a *Coronaviridae* infection, according to anyone of claims 1 to 12; which is for reducing the *Coronaviridae* viral load.

22. The compound or any one of its prodrugs or any one of its pharmaceutically acceptable salts, for use in a method for treating or preventing a *Coronaviridae* infection, according to anyone of claims 1 to 12; which is in combination with:
- a dynamin inhibitor, such as Dynasore; and/or
- an antibiotic, such as one selected from the group consisting of beta-lactams, fluoroquinolones, and macrolides; and/or
- an anti-inflammatory compound, such as one selected from the group consisting of: anti-TNF, Jak inhibitors, anti-IL6 antibodies, IL6 receptor antagonists.

23. A dynamin inhibitor for use in a method for treating or preventing a *Coronaviridae* infection; in particular for reducing a *Coronaviridae* viral load.

24. A dynamin inhibitor for use in a method for treating or preventing a *Coronaviridae* infection, according to claim 23; wherein the dynamin inhibitor is a phenothiazine, or any one of its pharmaceutically acceptable salts.

25. A dynamin inhibitor for use in a method for treating or preventing a *Coronaviridae* infection, according to claim 24; wherein the dynamin inhibitor is a phenothiazine selected from the group consisting of: chlorpromazines, pecazines, and perphenazines, or any one of their pharmaceutically acceptable salts.

26. A dynamin inhibitor for use in a method for treating or preventing a *Coronaviridae* infection, according to claim 25; wherein the dynamin inhibitor is a chlorpromazine selected from the group consisting of: acepromazine, chlorpromazine, cyamemazine, levomepromazine, oxomemazine, promazine, promethazine, triflupromazine, or any one of their pharmaceutically acceptable salts.

27. A dynamin inhibitor for use in a method for treating or preventing a *Coronaviridae* infection, according to claim 25; wherein the dynamin inhibitor is a pecazine selected from the group consisting of: mesoridazine, metopimazine, pecazine, thioridazine, or any one of their pharmaceutically acceptable salts.

28. A dynamin inhibitor for use in a method for treating or preventing a *Coronaviridae* infection, according to claim 25; wherein the dynamin inhibitor is a perphenazine selected from the group consisting of: carfenazine, fluphenazine, perazine, perphenazine, prochlorperazine, trifluoperazine, or any one of their pharmaceutically acceptable salts.

29. A dynamin inhibitor for use in a method for treating or preventing a *Coronaviridae* infection, according to claim 23; wherein the dynamin inhibitor is selected from the group consisting of: a phenothiazine, Iminodyn-17, Iminodyn-22, Chlorpromazine, Dynasore, long chain amines, long chain ammonium salts, dynoles, DD-6, desipramine, fluoxetine, reboxetine, fluphenazine, haloperidol, clozapine, methylene blue, or any one of their pharmaceutically acceptable salts.

30. A dynamin inhibitor for use in a method for treating or preventing a *Coronaviridae* infection, according to anyone of claims 23 to 29; in combination with a compound of formula (I) or (II), or any one of their prodrugs or pharmaceutically acceptable salts; wherein compound (I) and compound (II) are respectively of formula (I) and (II)

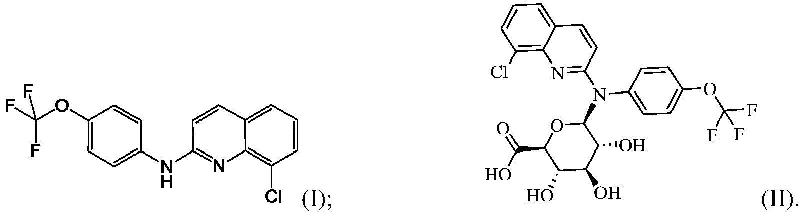

31. A dynamin inhibitor for use in a method for treating or preventing a *Coronaviridae* infection, according to anyone of claims 23 to 29; in combination with a cholesterol ester modulating agent.

32. A dynamin inhibitor for use in a method for treating or preventing a *Coronaviridae* infection, according to anyone of claims 23 to 29; in combination with chloroquine of hydrochloroquine.

33. A dynamin inhibitor for use in a method for treating or preventing a *Coronaviridae* infection, according to anyone of claims 23 to 29, in combination with at least one compound selected from (1) in combination with a compound of formula (I) or (II), or any one of their prodrugs or pharmaceutically acceptable salts; wherein compound (I) and compound (II) are respectively of formula (I) and (II) 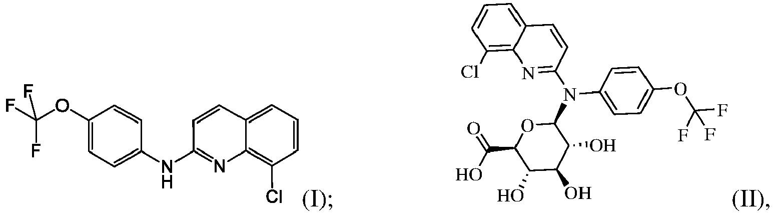 (2) a cholesterol ester modulating agent and (3) chloroquine or hydrochloroquine.

34. A pharmaceutical composition comprising a dynamin inhibitor or one of its pharmaceutically acceptable salts according to any one of claims 23 to 30; for use in a method for treating or preventing a *Coronaviridae* infection.

35. The pharmaceutical composition according to the preceding claim, further comprising at least one of compound (I) and (II), or any one of their prodrugs or pharmaceutically acceptable salts.
